# EUROPEAN PATENT SPECIFICATION

(11) **EP 1 276 424 B1**
(45) Date of publication and mention of the grant of the patent: **29.12.2004**
(21) Application number: 01923855.9
(22) Date of filing: 27.04.2001
(51) Int. Cl.: A61B 17/15

(54) **TENSER FOR PERFORMING A KNEE REPLACEMENT OPERATION**
SPANNGERÄT ZUM AUSFÜHREN EINER KNIEERSATZOPERATION
OUTIL CHIRURGICAL

(30) Priority: 27.04.2000 EP 00303551
(43) Date of publication of application: 22.01.2003
(73) Proprietor: Finsbury (Development) Limited, Leatherhead, Surrey KT22 0BA (GB)
(72) Inventor: TUKE, Michael, Antony, Guildford Surrey GU1 3TF (GB); WOZENCROFT, Robert, Michael, Epsom Surrey KT19 8SS (GB)
(74) Representative: Smaggasgale, Gillian Helen
(86) International application number: PCT/GB2001/001877
(87) International publication number: WO 2001/085038

(56) References cited:
- EP-A- 0 809 969
- US-A- 4 566 448
- US-A- 5 688 280
- US-A- 5 800 438
- US-A- 6 022 377

## Description

This invention relates to a surgical tool. In particular it relates to a tenser for use in performing a knee replacement operation.

The human knee is a complex joint formed between the femur and the tibia. Although the axis of the tibia is substantially vertical in an erect standing human, the femur shaft axis is usually at an angle of about 7° out of the vertical. This is to allow the weight of the upper part of the body to be transmitted from the acetabulum of the hip joint to the ball head at the top of the femur, which is offset from the femur shaft axis, in a straight line from the centre of the hip to the centre of the knee and then via the tibia in the same straight line to the centre of the ankle.

At the lower end of the femur there are formed two femoral condyles, namely a lateral condyle and a medial condyle. Corresponding generally concave lateral and medial depressions are formed on the upper end of the tibia. A layer of cartilage is interposed between the load bearing surfaces of the joint. A pair of collateral ligaments connect the femur to the tibia, one on each side of the knee joint. These collateral ligaments play an essential role in holding the knee joint together and in providing stability therefor. Thus in the natural knee they are taut throughout the range of motion of the knee joint as the person bends his or her knee in walking or running. Moreover the line joining the points of attachment of the collateral ligaments to the femur effectively acts as the axis about which the knee bends.

A further pair of cruciate ligaments extend parallel to a substantially sagittal plane of the knee joint between the medial and lateral condyles. The anterior cruciate ligament connects an anterior part of the top end of the tibia to a posterior surface of a recess at the bottom end of the femur at the rear of the knee joint. The posterior cruciate ligament is positioned adjacent the anterior cruciate ligament and connects a posterior part of the top of the tibia to another part of the same back surface of the afore-mentioned recess in the rear of the knee joint. The patella covers the front side of the knee joint.

The human knee can bend through an angle of approximately 160°. In the flexed condition of the knee a person can rotate his or her tibia about its axis relative to the femur through a small angle. However, in the straightened condition of the knee, the tibia becomes effectively locked with respect to the femur so that rotation of the tibia about its axis relative to the femur becomes essentially impossible.

With passage of time many people suffer wear of, or damage to, the bearing surfaces of one or both of the bones of the knee joint. In severe cases it is expedient to perform a knee replacement operation in which the worn or damaged bone is resurfaced. In such an operation part of the upper end of the tibia and/or the lower end of the femur may be cut away and a tibial and/or femoral component fitted as an implant. If necessary, a patella implant can also be fitted in the course of the implantation operation.

Various designs of knee prosthesis have been proposed. For example, a meniscal bearing total knee, designated the Dual Bearing Knee System (DBK), has been developed by Finsbury (Development) Limited of 13 Mole Business Park, Randalls Road, Leatherhead, Surrey KT22 0BA, United Kingdom, in conjunction with Osaka University Medical School of Osaka, Japan and Midland Medical Technologies Ltd., of Birmingham University Research Park, Vincent Park, Edgbaston, Birmingham B15 2SQ. This knee prosthesis comprises a metal femoral component, which is made in various sizes in either left side or right side form, a metal tibial component, a tibial insert of a plastics material, such as high density, high molecular weight polyethylene, and a patella component. The tibial component is provided in a series of sizes, designated T1 to T6. Similarly the tibial insert is provided in small, medium, and large sizes, each in a series of thicknesses, which correspond, for example, to a combined thickness of tibial implant and tibial insert of 9 mm, 11 mm, 13 mm, 15 mm or 17 mm respectively, with either a hole or a slot to receive a peg on the tibial component. The femoral component is provided in left side and right size forms in a series of sizes, designated F1 to F6, while the patella component is provided in a variety of sizes, for example, 23 mm, 26 mm, and 30 mm. Within these ranges of sizes and styles of components each can be matched with more than one size and/or style of the other components.

In a knee replacement operation using the DBK knee prosthesis it is usual to resect the tibia first in readiness for the tibial component. This comprises a plate with a locating pin on its underside which is received in a corresponding bore drilled in the tibia by the surgeon, after having first resected part of the top end of the tibia. On top of the horizontal plate is an upwardly projecting peg. The tibial insert has a corresponding cavity in its underside in which the peg fits, the arrangement permitting a certain amount of rotation of the tibial insert relative to the axis of the tibia after implantation. If the surgeon elects to preserve the posterior cruciate ligament, then a tibial insert with a slot in its underside will normally be used, this feature permitting the tibial insert both to rotate and to slide on the tibial component. On the other hand, the surgeon may elect to resect the posterior cruciate ligament, in which case it will normally be appropriate to use a tibial insert with a circular cavity in its underside so that in this case the tibial insert can only rotate on the tibial component. The top surface of the tibial insert has a pair of depressions in which the condyles of the femoral component bear. The femoral component has a groove in its front side in which a patella component can track.

After a knee replacement operation a patient will usually be able to bend the knee through a smaller angle than the natural knee, generally through about 120°.

Although the surgeon can make a single horizontal cut in resecting the tibia prior to implanting a knee prosthesis, the resection of the femur is much more complex and a total of as many as five saw cuts may be necessary to the femur, including an anterior cut and a posterior cut. These cuts must be made accurately and in an appropriate manner, bearing in mind the size and geometry of the femoral component, in order that the knee prosthesis can be implanted satisfactorily. It is particularly important that the collateral ligaments remain taut throughout the approximately 120° range of motion of the knee after the implantation operation in order that the knee shall be stable and function properly.

In any knee replacement operation the prosthesis is generally designed to copy as closely as possible the function of a healthy knee. In the course of such an operation various checks are made to ensure that the implanted components are aligned correctly with natural bone. For stability the aim is to implant a component of the same thickness as the bone which has been removed to make space for the implant. To achieve this the instruments conventionally used reference the articulating surfaces of the bone and position the saw cuts a certain distance from these surfaces. Most conventional instruments work according to this principle. However, the design of such instruments generally does not take into account the fact that the bone may already be severely worn, nor that a leg misalignment may be present or that the collateral ligaments may be too tight or too loose, a factor which may affect the range of motion of the knee or its stability.

These issues can be addressed after the bone cuts have been made, notably by checking the gaps between the resected bones both in flexion and in extension, as well as the leg alignment. However, at this stage of the operation, it can be difficult to make corrections and the operation can become very complicated.

An example of a device for measuring bone gaps during a knee replacement operation is described, for instance, in United States Patent No. 5,800,438.

An alternative technique involves applying a tension in the knee and cutting the correct amount of bone to maintain this tension when the knee replacement components are in place. This technique is often called the tenser or tensor technique and generally provides greater accuracy. It takes into account the presence of the collateral ligaments and the relationships between the tibia and the femur. The instruments used in such a technique are known as tensers or tensors. Such instruments conventionally comprise a handle with a pair of parallel paddles, one fixed and the other movable relative to the fixed paddle. In an alternative design there is a single fixed paddle and a pair of independently movable paddles. In either case, with the paddles closed up one to another, the surgeon can insert them into the knee joint, having first resected the tibia, before moving the paddles apart until the collateral ligaments are tensioned and thereby measuring the distance between the upper surface of the tibial implant and the as yet unresected femur, both in the flexed and straightened condition of the knee. The measurements thus obtained provide assistance to the surgeon in deciding where to make the anterior and posterior cuts.

European Patent Publication No. 0 809 969 A2 describes a bone cutting guide for use in the tenser technique with a fixed paddle and a movable paddle for fitting in a gap between a tibia and a femur during surgery of the knee. This guide has a body with a handle at its posterior end and with a first tissue engaging member in the form of the fixed paddle at its anterior end. The undersurface of the fixed paddle member in use of the guide is placed on a resected surface of a tibia which extends substantially orthogonally with respect to the axis of the tibia. A first movable member in the form of a shaft carrying the teeth of a rack is slidable in a bore in the body in a direction which is substantially perpendicular to the undersurface of the fixed paddle. This shaft can be moved relative to the body and the fixed paddle by a lever in the form of a key attached to a pinion which is mounted in the body and which engages with the rack so that the upper surface of the movable paddle can be placed against the femur. The guide also includes a pair of support members in the form of guide shafts which can slide in respective bores in the body parallel to the rack-carrying shaft (and hence in a direction substantially perpendicular to the undersurface of the fixed paddle). One of these guide shafts is provided with a friction locking means in the form of a fine rack on its inner side which is engaged by a resilient element located within the corresponding bore in the body. The guide shafts carry a pair of second movable members in the form of a drill guide block fixed to each guide shaft so that movement of the guide shafts in the body also causes the drill blocks to move in a direction substantially perpendicular to the undersurface of the fixed paddle. The rack-carrying shaft carries a third movable member in the form of a rotatable shaft at its upper end which can pivot through 5° in either direction and the movable paddle is attached to this rotatable shaft. This same rotatable shaft has a square section opening for passage of a square guide on an intramedullary rod.

Although existing forms of tenser provide a degree of assistance to a surgeon in judging where to resect the femur of a patient undergoing a knee implantation operation, there exists a need to provide a surgical instrument which will enable the position of the saw cuts to be determined with greater accuracy. There is also a need to provide a tenser which facilitates the choice of size, style, or both of the femoral component and/or the tibial insert by a surgeon in the course of an implantation operation so as to provide an optimum knee replacement for the patient. In addition there exists a need for an improved tenser which will enable the surgeon to determine readily the existence of a leg misalignment and to correct the same. It would also be desirable to provide a tenser which enables a surgeon to put the collateral ligaments under a predetermined amount of tension during a knee replacement operation so that both when the knee is flexed and also when it is extended the collateral ligaments can in each case be put under the same amount of tension.

It is accordingly an object of the present invention to provide an improved form of tenser for use during a knee implantation operation.

It is a further object of the invention to provide a tenser which will enable the surgeon to determine with great accuracy where the saw cuts should be made during resection of a femur during a surgical operation to implant a femoral component of a knee prosthesis.

It is a still further object of the present invention to provide a tenser which will facilitate the choice by the surgeon of the size, of the shape or of both the size and the shape of the femoral component and/or of the tibial insert to be implanted so as to provide a successful knee replacement for a given patient.

It is an additional object of the invention to provide a tenser which is adapted in use to put the collateral ligaments under a predetermined amount of tension during a knee replacement operation whether the knee is flexed or extended and to subject the collateral ligaments in each case to the same amount of tension.

According to the present invention there is provided a tenser for use during implantation of a knee implant comprising:
a body having a handle at its posterior end and a fixed paddle member at its anterior end, the fixed paddle member having an undersurface defining a plane and adapted for positioning on a resected surface of a tibia which extends substantially orthogonally with respect to the axis of the tibia;
a first movable member movably mounted to the body for movement in a direction substantially perpendicular to the plane of the undersurface of the fixed paddle member and having a movable paddle member mounted thereon;
an operating lever mounted in the body and operatively coupled to the first movable member for moving the first movable member relative to the body to move the movable paddle member relative to the fixed paddle member and into engagement with a femur during an operation to implant a knee implant;
a support member adjustably mounted to the body for movement in a direction substantially perpendicular to the plane of the undersurface of the fixed paddle member;
first locking means for locking the support member to the body;
a second movable member adjustably mounted to the support member for movement in a direction substantially perpendicular to the plane of the undersurface of the fixed paddle member, the second movable member being provided with a slot for passage of a surgical saw for making an anterior cut during resection of a femur in preparation for implantation of a femoral component;
second locking means for locking the second movable member to the support member; and
a third movable member slidably mounted to the body for movement in a direction substantially perpendicular to the plane of the undersurface of the fixed paddle member and having guide means for passage of an intramedullary member for insertion in a prepared intramedullary bore in a femur to be resected.

In a preferred form of tenser according to the invention the fixed paddle member has a planar undersurface. However, exact planarity of the undersurface of the fixed paddle member is not essential. Thus if the undersurface is non-planar (and has, for example, a shallow wave-like cross section defining a pair of waves), the lowest points of the undersurface which are adapted, in use, to contact the resected surface of the tibia will, for the purposes of the invention, define the plane of the undersurface of the fixed paddle member.

Preferably the operating lever is pivotably mounted in the body and is spring loaded so as to bias the first movable member in an opening direction to cause the movable paddle member to move away from the fixed paddle member. Moreover the handle may be provided with a longitudinal bore which houses a compression spring which is arranged so as to bias the operating lever in the opening direction. A piston may be disposed in the bore and interposed between an anterior end of the compression spring and the operating lever. At least a part of the longitudinal bore is preferably threaded, and a threaded member is threadedly engaged in the threaded part of the bore, while a posterior end of the compression spring is arranged to bear against the threaded member, whereby the compression in the compression spring can be adjusted by moving the threaded member along the threaded part of the longitudinal bore. In such a tenser the handle may carry at its posterior end a compression adjustment means adapted for moving the threaded member along the threaded part of the longitudinal bore, the compression adjusting means including torque limiting means adapted to limit the torque which can be applied by the compression adjustment means to move the threaded member along the threaded part of the bore. Moreover the compression adjustment means may comprise an adjustment knob rotatable about a longitudinal axis of the handle, an adjustment member in the longitudinal bore and rotatable about the longitudinal axis of the handle, the adjustment member being engageable with the threaded member to move the threaded member along the threaded part of the bore, and spring loaded clutch means connecting the adjustment knob and the adjustment member, whereby upon applying a torque to the adjustment knob in excess of a predetermined torque the spring loaded clutch means prevents further rotation of the adjustment member to increase the compression in the compression spring. The adjustment knob may have a compression adjustment member threadedly engaged therewith and the spring loaded clutch means may comprise a secondary compression spring which is trapped between the compression adjustment member and the adjustment knob and whose compression can be varied by adjusting the position of the compression adjustment member relative to the adjustment knob so as to vary the value of the predetermined torque. Preferably the spring loaded clutch means comprises a collar on the adjustment member having on its posterior side a plurality of radial ribs and the adjustment knob is formed with a corresponding plurality of radial grooves on its anterior side for engagement with the radial ribs.

In a preferred form of tenser the body bears first indicia corresponding to the thicknesses of available sizes of tibial insert, and the support member bears a second indicium adapted to indicate against a selected first indicium an appropriate thickness of tibial insert for implantation.

It is also preferred that the support member is provided with a window, and that the body bears a third indicium which is adapted to be visible through the window over a part only of the available range of movement of the support member relative to the body and which is adapted to indicate, when the third indicium is visible in the window , that it is appropriate to select a thickness of tibial insert. In addition the second movable member can be provided with fourth indicia corresponding to available sizes of femoral implant and adapted to indicate by reference to an indicium on the support member an appropriate size of femoral implant to use with a selected thickness of tibial insert.

The tenser of the invention preferably further includes a stylus or pointer device adapted for mounting on the second movable member for contacting a shaft of a femur to be resected during the knee implantation operation. In such a tenser the second movable member may include an arm adapted to project forwardly over the condyles of a femur to be resected and the arm may carry a quick release locking device for detachably securing the stylus or pointer to the second movable member.

The third movable member preferably carries an apertured block which is rotatable relative to the third movable member about an axis which is substantially perpendicular to the plane of the undersurface of the fixed paddle member, while the guide means includes at least one aperture in the apertured block whose axis lies in a plane substantially parallel to the plane of the undersurface of the fixed paddle member, whereby the axis of the aperture can be aligned with the axis of an intramedullary bore drilled in a femur to be resected by rotation of the apertured block about its axis. Furthermore the third movable member can be provided with a ratchet arc substantially coaxial with the axis of rotation of the apertured block and a lever with a spring loaded locking device having a detent engageable with the ratchet arc can be coupled to the apertured block, whereby the apertured block can be locked at a selected position to prevent further rotation about its axis of rotation.

It is also preferred in a tenser according to the invention to include further a leg alignment member which can be operatively coupled to the tenser for checking the alignment of the tenser relative to the axis of a femur to be resected. In one form the leg alignment member comprises an elongate jogged member and is provided with a transverse peg, while the body of the tenser is provided with a corresponding transverse bore to receive the peg, whereby an anterior portion of the leg alignment member can be positioned forwardly of the tenser along a sagittal plane of the tenser for overlying the ball head of a femur to be resected. In an alternative form the leg alignment member comprises an elongate member and the tenser further includes a support member for the leg alignment member which can be mounted to the support member, whereby an anterior portion of the leg alignment member can be positioned forwardly of the tenser along a sagittal plane of the tenser for overlying the ball head of a femur to be resected.

The tenser may further include a removable block which is engageable with the second movable member and is adapted for temporary securement to a femur to be resected. Such a removable block may be formed with a keyway for engagement with the second movable member. Moreover the tenser may further include a mounting means for detachable securement to the removable block and for detachably mounting a distal saw cut block thereto, the distal saw cut block being formed with a slot adapted for receipt of a surgical saw to make a distal cut in a femur to be resected. In one form the mounting means comprises a T-bar attachment including a bar engageable with the removable block and a crosspiece adapted to extend transverse to the axis of a femur to be resected when the mounting means is engaged with the removable block and the removable block is secured to the femur to be resected. The bar of such a T-bar attachment may be adapted to be received in a keyway in the removable block.

It is also preferred that the movable paddle member is pivotably mounted on the first movable member so as to be pivotable about an axis which is substantially parallel to the plane of the undersurface of the fixed paddle member and lies is a substantially sagittal plane of the tenser.

In addition the tenser of the invention preferably further includes a saw cut block for applying to a partially resected femur having an anterior saw cut and a distal saw cut formed therein, the saw cut block being provided with slots adapted for making a posterior saw cut, a first oblique saw cut, and a second oblique saw cut.

In order that the invention may be clearly understood and readily carried into effect a preferred embodiment thereof will now be described, by way of example only, with reference to the accompanying drawings, wherein:
Figure 1 is an exploded perspective view of a tenser constructed in accordance with the invention;
Figure 2 is a partial longitudinal section through the tenser of Figure 1;
Figure 3 is a further longitudinal section through part of the tenser of Figures 1 and 2;
Figure 4 is a cross section on the line IV-IV of Figure 3;
Figure 5 is a perspective view of one of the parts of the tenser of Figures 1 to 4;
Figure 6 is a perspective view of the partially assembled tenser of Figures 1 to 5;
Figure 7 is a side view of the partially assembled tenser of Figure 6 in combination with a femoral component of a total knee replacement joint;
Figure 8 is a top plan view of the partially assembled tenser of Figures 6 and 7;
Figure 9 is a view from the rear of the partially assembled tenser of Figures 6 to 8;
Figure 10 is a perspective view of a leg alignment tool for use with the tenser of Figures 1 to 9;
Figure 11 is a diagrammatic view of a tibial insert and femoral component of a knee implant in the flexed position of the knee after implantation;
Figure 12 is a corresponding diagrammatic view of the tibial insert and femoral component of Figure 11 with the knee implant in the extended position of the knee;
Figure 13 is a diagrammatic front view of the femur and tibia of an erect healthy human;
Figure 14 illustrates the position on a tibia of the line of resection as a first step in implanting a knee implant;
Figure 15 illustrates a resected tibia and a resected femur in the flexed condition of the knee;
Figure 16 illustrates the resected tibia and resected femur of Figure 15 in the extended position of the knee;
Figure 17 shows how a intramedullary bore is offset from the straight line joining the centre of the human hip, the centre of the knee, and the centre of the ankle;
Figure 18 shows an intramedullary member for use with the tenser of Figures 1 to 10 in position in an intramedullary bore drilled in a patient's knee;
Figure 19 illustrates a first stage in implantation of a femoral component of a knee implant in a patient with an already resected tibia and with an intramedullary bore already drilled in the patient's femur;
Figure 20 illustrates the next step in the implantation procedure;
Figure 21 is a perspective view of the tenser of Figures 1 to 10 being prepared for the next step in the implantation operation;
Figure 22 is a diagrammatic top plan view of the tenser of Figures 1 to 9 with an alternative form of leg alignment tool in position in readiness for the next step by the surgeon;
Figure 23 shows the stylus of the tenser of Figures 1 to 9 being positioned on a patient's femur;
Figure 24 is a side view of the tenser of Figures 1 to 9 in position for the next stage of the implantation operation;
Figure 25 is a rear view of the tenser of Figure 24 showing one possible scenario for the next stage of the implantation operation;
Figure 26 is a side view of the tenser of Figures 1 to 9 being positioned on another patient's femur;
Figure 27 is a rear view of the tenser shown in Figure 26;
Figure 28 shows how one combination of tibial insert and femoral component represents a good fit for the patient of Figure 23;
Figure 29 illustrates the positions of bone cuts during resection of the femur of the patient of Figure 23;
Figure 30 shows an alternative combination of tibial insert and femoral component for the patient of Figure 26;
Figure 31 illustrates the positions of bone cuts during resection of the femur of the patient of Figure 26;
Figure 32 shows the anterior cut being made during resection of a patient's femur;
Figure 33 is a perspective view of a block to be used in making further cuts during resection of the patient's femur;
Figure 34 shows the next stage in resecting the patient's femur;
Figure 35 shows the patient's femur with the block of Figure 33 in place;
Figure 36 is a front view of the patient's knee in the extended position with the block of Figure 33 in place in readiness for the next step of the surgical procedure;
Figure 37 is a side view of the knee of Figure 36;
Figure 38 is a similar view with the tenser reinserted;
Figure 39 is a front view of a T-bar attachment for securing on the block of Figure 33;
Figure 40 is a rear view of the reinserted tenser in the knee of the patient of Figure 23;
Figure 41 is a side view of the T-bar attachment of Figure 39;
Figure 42 is a front view of a guide for making the distal cut during resection of a patient's femur;
Figure 43 is a side view corresponding to Figure 40;
Figure 44 is a rear view of the reinserted tenser in the knee of the patient of Figure 26;
Figure 45 is a front view of the guide of Figure 42;
Figure 46 is a side view corresponding to Figure 44;
Figure 47 is a front view corresponding to Figure 40 of a patient with a misaligned leg;
Figure 48 is a similar front view of the leg of another patient with another form of misaligned leg;
Figure 49 is a perspective view of a block for effecting the distal cut during resection of a patient's femur;
Figure 50 is a side view of a patient's leg with the block of Figure 33 and the T-bar attachment of Figure 39 in place on the patient's femur;
Figure 51 illustrates the making of the distal cut during resection of a patient's femur;
Figure 52 illustrates the making of the remaining cuts during resection of a patient's femur;
Figure 53 illustrates the fitting of the femoral component of the knee implant to the resected femur of the patient; and
Figure 54 is a perspective view of a tool for adjustment of the tenser of Figures 1 to 9.

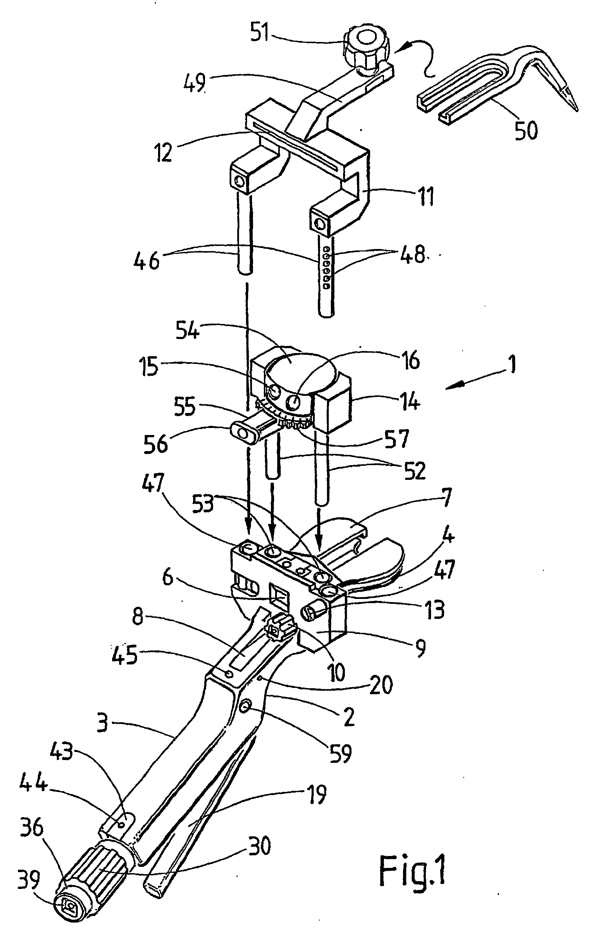

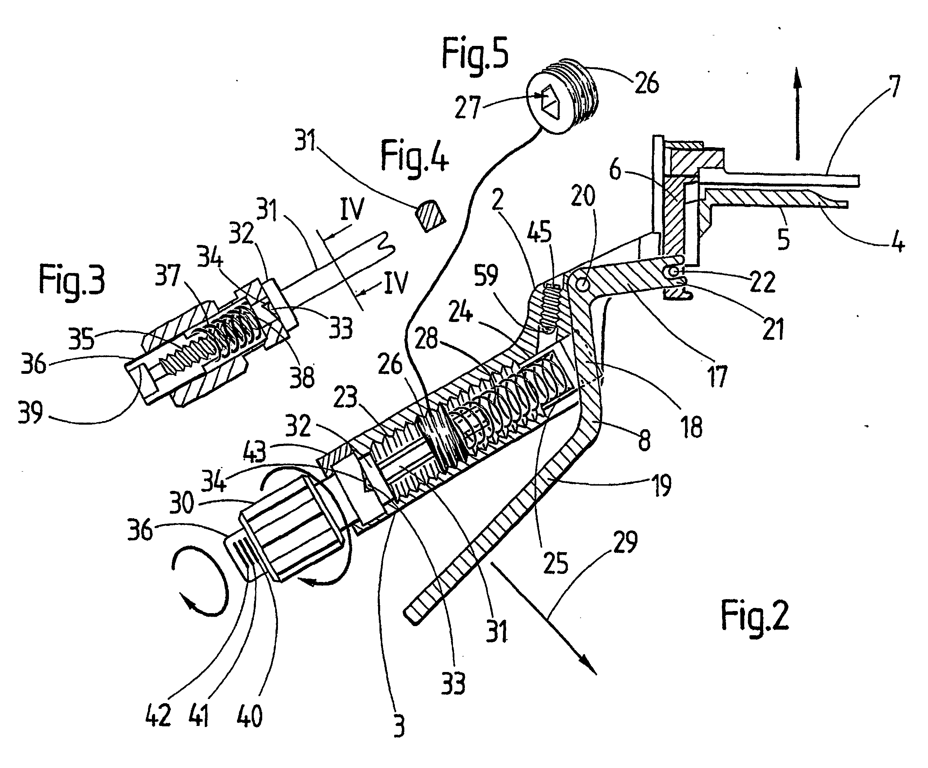

Referring to Figure 1 of the drawings, a tenser 1 for use during implantation of a knee implant comprises a body 2 having a handle 3 at its posterior end and a fixed paddle member 4 at its anterior end. The fixed paddle member 4 has an undersurface 5 (see Figure 2) for positioning on a resected surface of a tibia. This resected surface will normally be intended to extend substantially orthogonally with respect to the axis of the patient's tibia. A first movable member 6 is movably mounted in the body 2 for movement in a direction substantially perpendicular to the undersurface 5 of the fixed paddle member 4 and has a movable paddle member 7 mounted thereon. Movable paddle member 7 is pivotably mounted on first movable member 6 for limited pivotal movement about an axis which lies in the sagittal plane of the body 2 and substantially parallel to the plane of the undersurface 5 of fixed paddle member 4. Movable paddle member 7 can thus tilt to either side from a first position in which it extends substantially parallel to the fixed paddle member 4 and can be locked in one of two positions, one in which it is tilted 3° from the first position to one side and the other in which it is tilted through a similar angle to the other side, by means of a pin (not shown in Figure 1) which is inserted in a corresponding hole (also not shown in Figure 1) in first movable member 6.

A spring loaded operating lever 8 is mounted in the body 2 and is operatively coupled to the first movable member 6 for moving the first movable member 7 relative to the body 2 to move the movable paddle member 6 relative to the fixed paddle member 4 and into engagement with a femur during an operation to implant a knee implant.

A support member 9 is adjustably but captively mounted on the body 2 for movement in a direction substantially perpendicular to the undersurface 5 of the fixed paddle member 4. A first locking means in the form of a locking knob 10 is provided for locking the support member 9 to the body 2.

A second movable member 11 is adjustably mounted in the support member 9 for movement in a direction substantially perpendicular to the undersurface 5 of the fixed paddle member 4. This second movable member 11 is provided with a slot 12 for passage of a surgical saw for making an anterior cut during resection of a femur in preparation for implantation of a femoral component of a knee implant. A second locking means in the form of a spring ball grub screw 13 is provided for locking the second movable member 11 to the support member 9.

A third movable member 14 is slidably mounted in the body 2 for movement in a direction substantially perpendicular to the undersurface 5 of the fixed paddle member 4 and has guide means in the form of apertures 15, 16, each adapted for passage of an intramedullary member (not shown in Figure 1) for insertion in a prepared intramedullary bore in a femur to be resected.

As can be seen from Figure 2, operating lever 8 has two arms 17 and 18 in the form of a bell crank lever with a posterior extension 19 formed on arm 18. It is arranged to pivot about a pivot pin 20 fixed in body 2. At its anterior end arm 17 has a bifurcation 21 which is arranged to engage a pin 22 mounted on first movable member 6 so that, as operating lever 8 is pivoted about pivot pin 20, first movable member 6 is caused to move substantially perpendicular to undersurface 5 of fixed paddle member 4.

Handle 3 has a threaded bore 23 that extends longitudinally thereof. This communicates at its anterior end with a smooth bore 24 in which a cup shaped piston 25 is arranged to slide. A threaded member 26 with an axial keyway 27 (see Figure 5) is threadedly engaged in threaded bore 23 and serves to retain a compression spring 28 within threaded bore 23. Spring 28 bears on threaded member 26 and biases piston 25 towards the anterior end of body 2, while piston 25 5 in turn bears on arm 18 of operating lever 8, thereby biasing it in the direction of arrow 29.

At its posterior end handle 3 carries an adjustment knob 30 by means of which the compression in compression spring 28 can be adjusted. This adjustment knob 30 is held captive on an adjustment member 31 which extends towards the anterior end of the handle 3 and has a cross section (see Figure 4) which enables it to pass through keyway 27 (see Figure 5) and matingly engage therewith. Adjustment member 31 has a collar 32 integrally formed therewith and a pair of radial ribs 33 are formed on the posterior side of collar 32. Adjustment knob 30 is formed at its anterior end with a corresponding pair of radial grooves 34 which can matingly engage with ribs 33 on collar 32 to effect rotation of adjustment member 31. The posterior end 35 of adjustment member 31 is externally screw threaded (see Figure 3) and is threadedly engaged with a compression adjustment member 36 which bears on a compression spring 37 that is trapped between compression adjustment member 36 and a washer 38. Compression spring 37 together with radial ribs 33 and radial grooves 34 thus act as a form of spring loaded clutch between adjustment knob 30 and adjustment member 31.

Compression adjustment member 36 is formed at its posterior end with a square section recess 39 for reception of a square section key (not shown in Figure 3) by means of which compression adjustment member 36 can be screwed further onto or off screw threaded end 35 of adjustment member 31. By adjusting the position of compression adjustment member 36 with respect to adjustment knob 30, the compression in spring 37 can be adjusted. This in turn will vary the force preventing grooves 34 riding over ribs 33 when adjustment knob 30 is rotated to move threaded member 26 along threaded bore 23 to increase the compression in spring 28. Hence, since the compression in spring 37 limits the torque which can be applied by means of adjustment knob 30 via grooves 34 5 and ribs 33 to adjustment member 31, the combination formed by compression adjustment member 36, spring 37, and adjustment knob 30 acts as a torque limiter.

As can be seen from Figure 2, compression adjustment member 36 can bear indicia 40, 41 and 42 indicating the compression to be applied to the spring 28, and hence (as will be described further below) to the collateral ligaments in use of the tenser 1. Such indicia may indicate, for example, 26 kg, 28 kg, and 30 kg respectively. By varying the position of compression adjustment member 36 relative to adjustment knob 29, the surgeon can vary the tension to be applied to the collateral ligaments during use of the tenser 1, as will be explained in more detail hereinbelow.

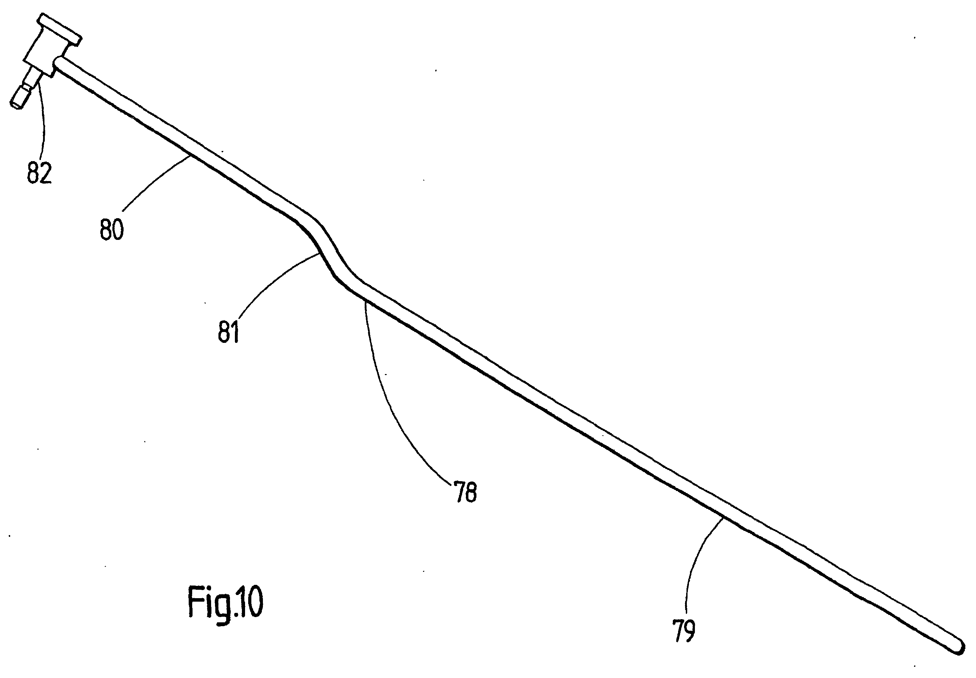

A retainer member 43 and a retaining screw 44 retain adjustment knob 30 in place on handle 3. A tapped hole 45 is provided for a spring ball grub screw (not shown) for locking a leg alignment member (which will be described further below in relation to Figure 10).

Second movable member 11 is formed with a pair of rods 46 which are arranged to slide in corresponding bores 47 in support member 9. One of the rods 46 is formed with a series of indentations 48 for engagement by a ball on the end of spring ball grub screw 13. Second movable member 11 also carries a forwardly projecting cantilevered arm 49 to whose anterior end can be fitted a stylus or pointer 50. This can be secured in place with the aid of a quick locking knob 51.

Third movable member 14 is provided with a pair of rods 52 which are received in bores 53 in body 2. Rods 52 can be of different diameters so as to ensure that third movable member 14 can only be assembled onto body 2 in the correct orientation. Third movable member 14 is retained in tenser 1 by means of second movable member 11 but can still slide to a limited extent in body 2 in a direction substantially perpendicular to the undersurface 5 of fixed paddle member 4. It carries a captive block 54 which is rotatable through an angle of up to about 10° about an axis substantially perpendicular to the undersurface 5 of fixed paddle member 4.

Rotation of block 54 can be effected by means of an adjustment lever 55 fixed to block 54. Adjustment lever 55 carries at its posterior end a spring loaded operating knob 56 which is arranged to cooperate with the teeth of a fixed ratchet arc 57 to lock block 54 relative to third movable member 14. Bores 15 and 16 are formed in block 54 and their axes are offset one to the left and the other to the right of the axis of adjustment lever 55. Moreover the axis of each of bores 15 and 16 intersects with the axis of rotation of block 54. By use of the lever 55 to rotate block 54, the bore 15 or 16 can either be aligned in the sagittal plane of tenser 1 or offset by an angle of up to 10° to the right or left respectively of this sagittal plane, for a purpose which will be explained further below.

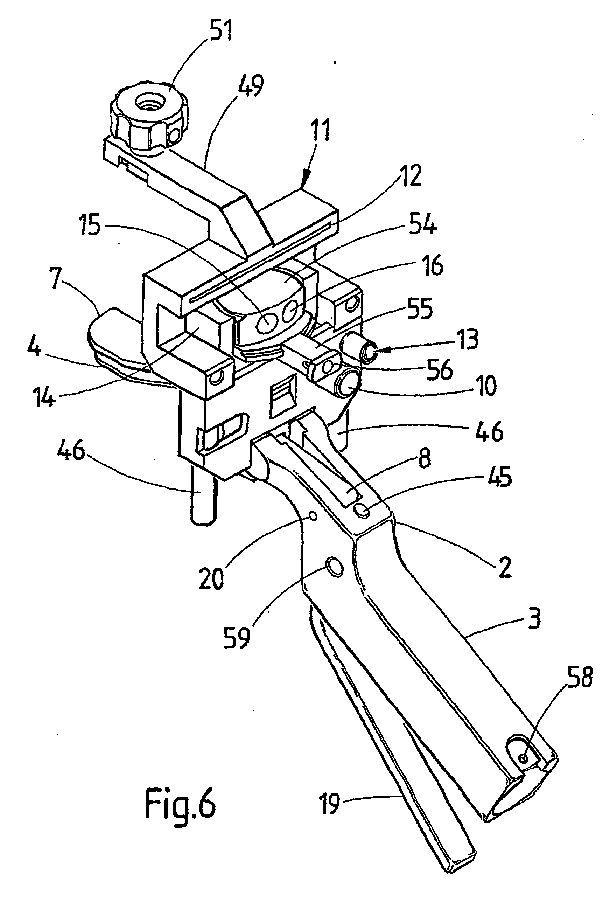

Referring now to Figure 6 of the drawings, reference numeral 58 indicates a threaded bore to receive retainer screw 44. Also visible in Figures 1 and 6 is a smooth bore 59 to receive a leg alignment rod (not shown in Figure 1 or in Figure 6) the purpose of which will be explained further below.

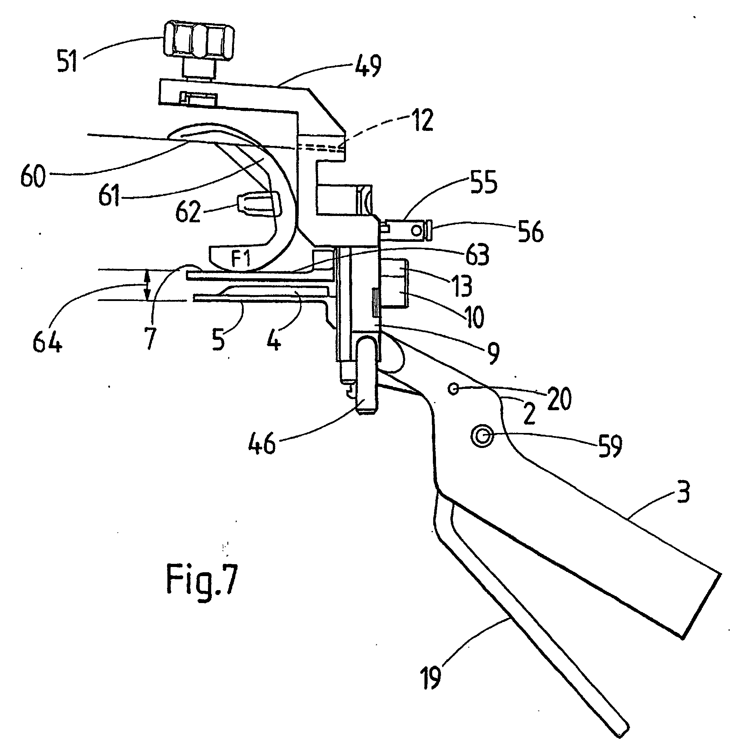

Figure 7 illustrates how the slot 12 can be aligned with a corresponding anterior surface 60 of a femoral component 61 of a knee implant. Femoral component 61 has a pair of locating pegs 62, one corresponding to each condyle. Assuming that the anterior cut and the other cuts are correctly made (as will be described further below) in the femur of the patient in whose knee the implant is being implanted, the illustrated femoral component 61 will be appropriate for use with a tibial implant (not shown) and a tibial insert (also not shown) whose combined thickness equals the distance between the upper surface 63 of movable paddle member 7 and the undersurface 5 of fixed paddle member 4. This distance is indicated at 64 in Figure 7.

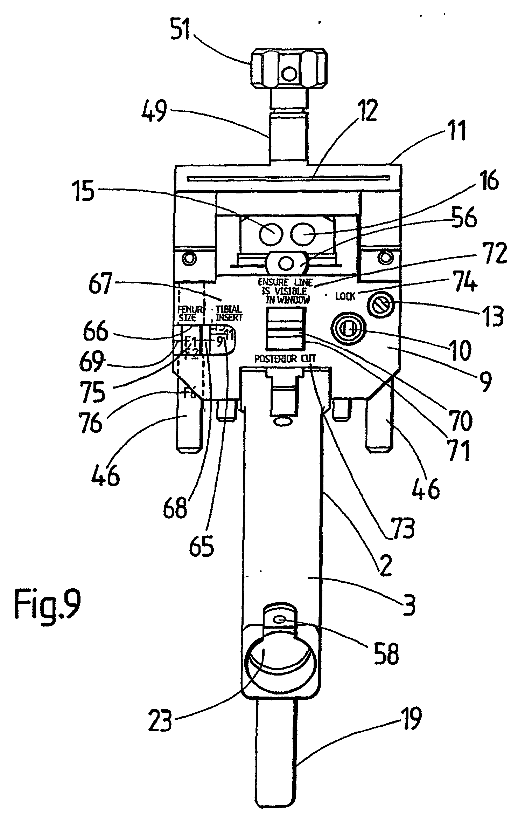

Referring now to Figure 9 of the drawings, body 2 carries a first series of indicia 65, each corresponding to a possible thickness of a combination of a tibial insert and tibial component. The tibial component will be of constant thickness while the surgeon will have available a series of tibial inserts of different thicknesses which differ from one another by 2 mm increments. Hence indicia 65 may, for example, indicate combinations of a tibial insert and of a tibial component of combined thickness 9 mm, 11 mm, 13 mm, 15 mm, and 17 mm. The indicia 65 are visible through a cut out window 66 in the back of support member 9. The support member 9 is itself inscribed with the legend "TIBIAL INSERT" as indicated at 67. In addition, support member 9 carries a pair of short lines 68 and 69 for a purpose to be described below.

First movable member 6 also carries an indicium 70 which indicates, as will be described below, the position for making a posterior cut in the patient's femur. Support member 9 should be adjusted so that indicium 70 is visible in a window 71 cut in the back of support member 9 before any cuts are made in the patient's femur. Support member 9 also carries a legend, indicated at 72, reading "ENSURE LINE IS VISIBLE IN WINDOW", and a further legend "POSTERIOR CUT" as shown at 73. A further legend "LOCK" is positioned adjacent spring ball grub screw 13 on support member 9, as indicated by reference numeral 74.

Second movable member 11 also carries on one of the rods 46 a series of indicia "F1" to "F6" as indicated by reference numerals 75 and 76. Only the indicia "F1", "F2" and "F6" are visible in Figure 9. These indicia 75 and 76 correspond to the different sizes of femoral component provided by the manufacturer of the knee implant. A legend 77 reading "FEMUR SIZE" is positioned on support member 9 adjacent window 66 as a reminder to the surgeon of the significance of the indicia 75 and 76 visible in the window 66.

Figure 10 shows a leg alignment member 78 for use with the tenser 1. This has a straight portion 79 which is connected to another straight portion 80 by means of a bent section 81 so as to form a jogged shaft. At one end it has a transverse peg 82 which is adapted for receipt in bore 59. When peg 82 is properly inserted in bore 59 the straight portion 79 is aligned with the sagittal plane of the tenser 1.

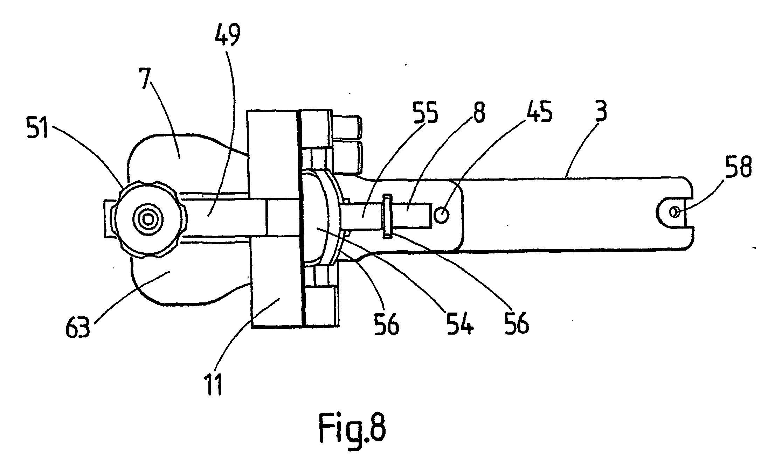

Other ancillary items of equipment for use with the tenser 1 of Figures 1 to 9 will now be described.

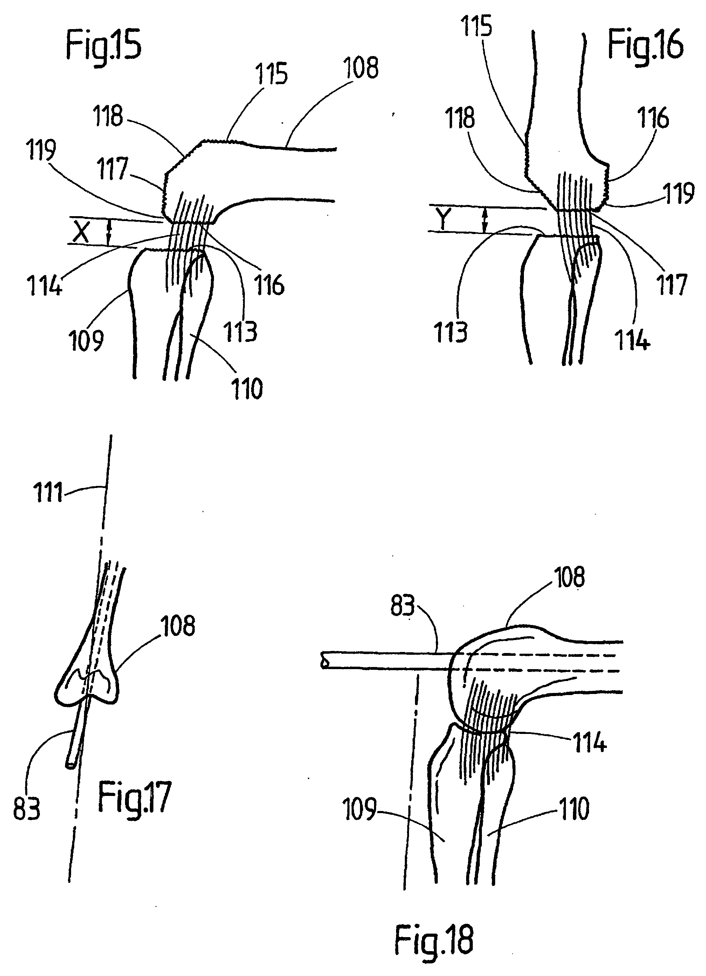

In Figure 17 there is indicated part of an intramedullary member 83 for insertion in a prepared intramedullary bore in the patient's femur.

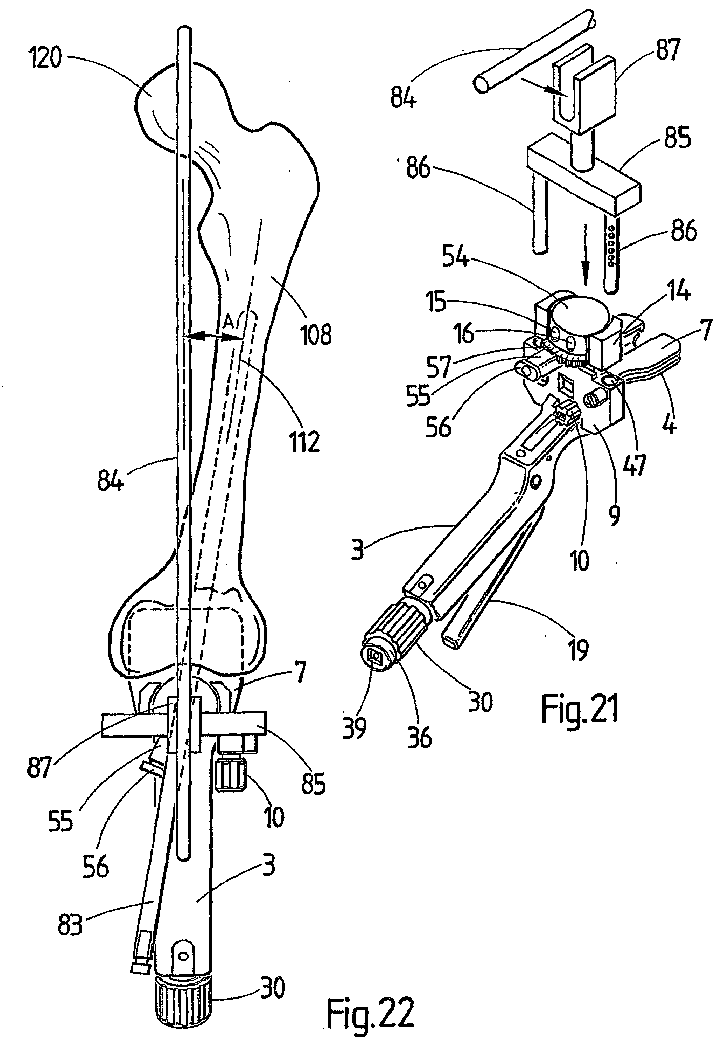

Figure 21 shows an alternative form of leg alignment member 84 and an external alignment rod holder 85. This has a pair of rods 86 which are adapted to be received in bores 47 in support member 9, as well as a support stirrup 87 for leg alignment member 84.

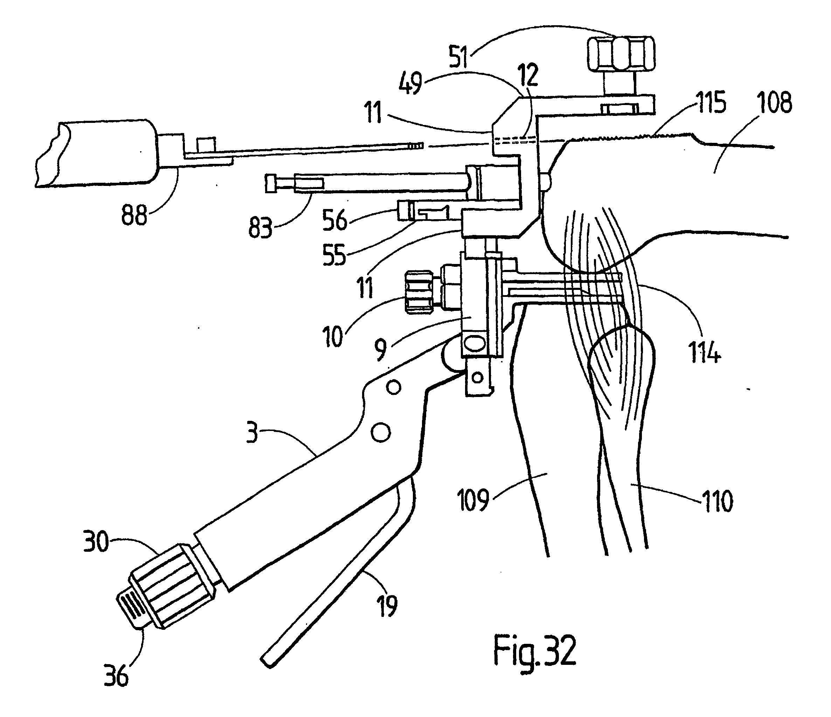

In Figure 32 there is shown an air operated surgical saw 88.

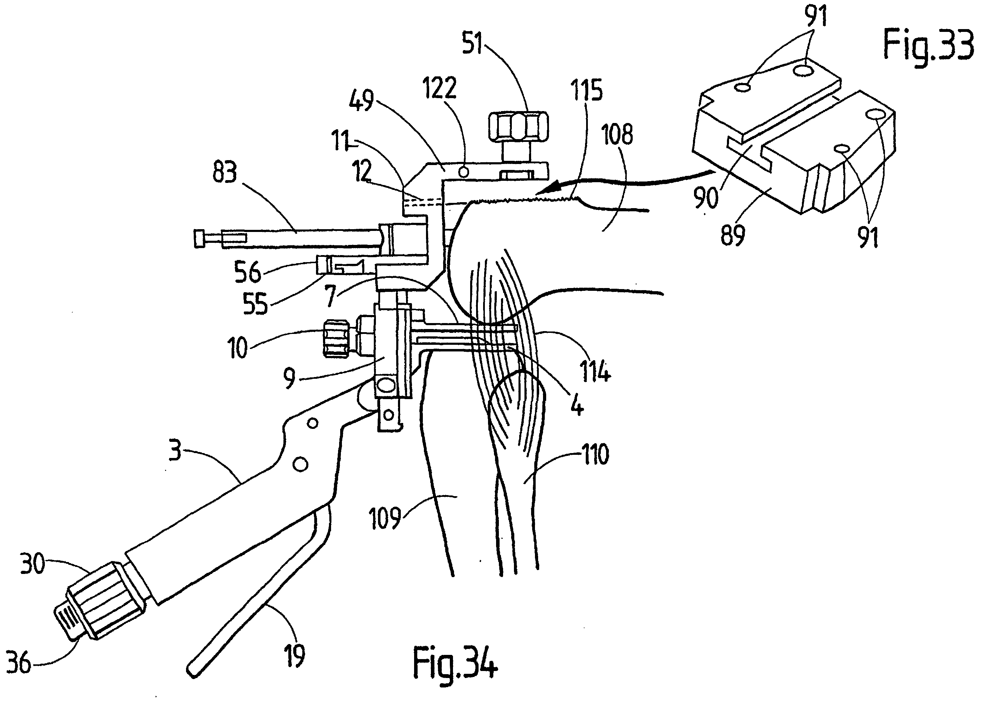

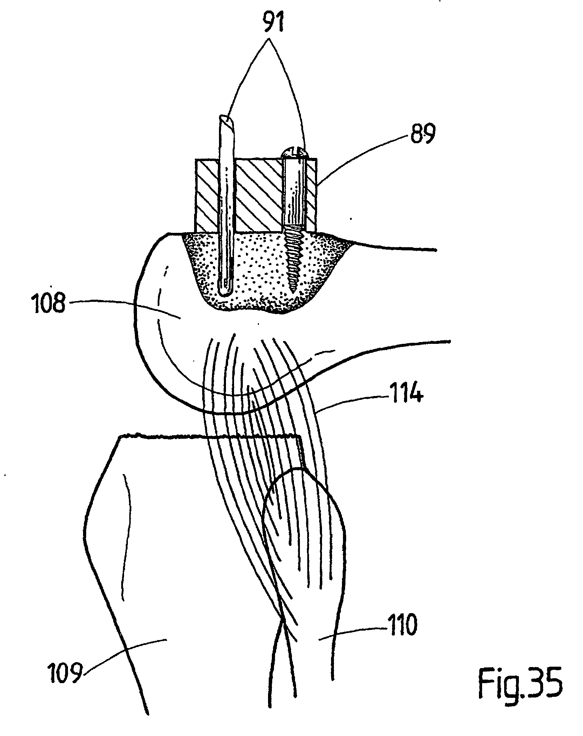

Figure 33 illustrates a block 89 which is intended for temporary securement to a patient's femur during a surgical operation to implant a knee implant. This is provided with a keyway 90 and with holes 91 for passage of pins or screws 92 (see Figure 35) for effecting temporary securement of the block 89 to a patient's femur.

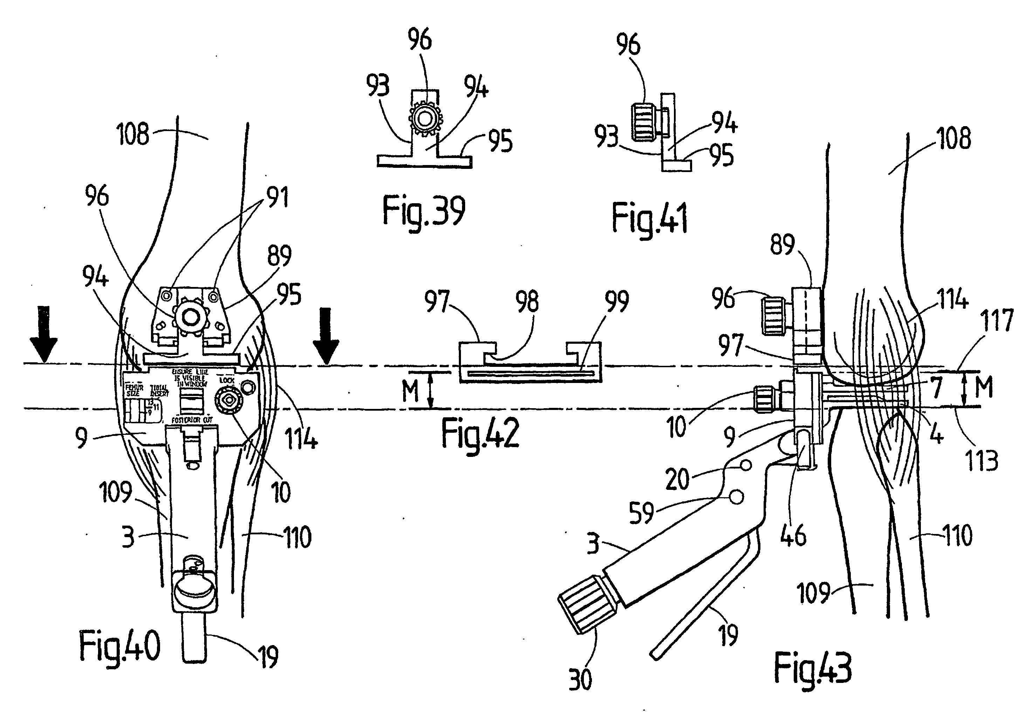

A T-bar attachment 93 is shown in Figures 39 and 41. This includes a bar 94 for receipt in keyway 90 of block 89 and a crosspiece 95. Quick release knob 96 is provided to enable T-bar attachment 93 to be removably secured to block 89.

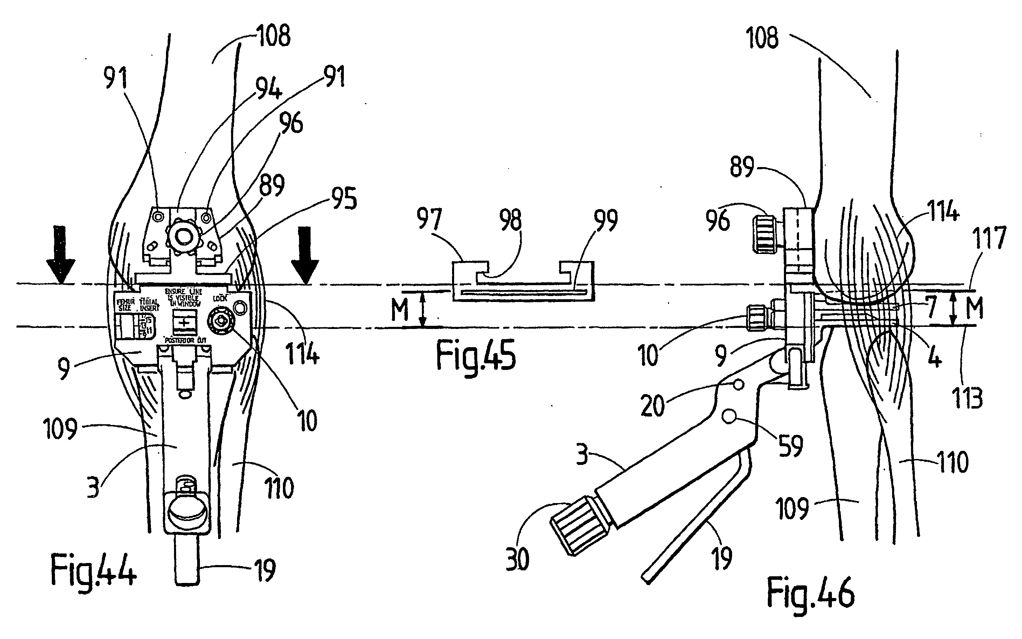

Figures 42 and 45 show a distal cutting block 97 with a keyway 98 that fits on crosspiece 95 and with a slot 99 for receipt of surgical saw 88 during the making of the distal cut in the course of resecting a patient's femur.

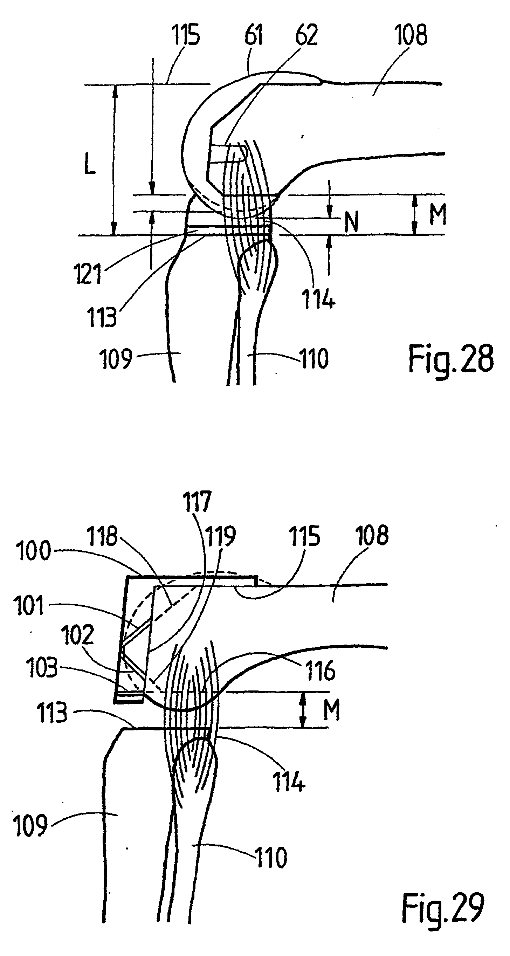

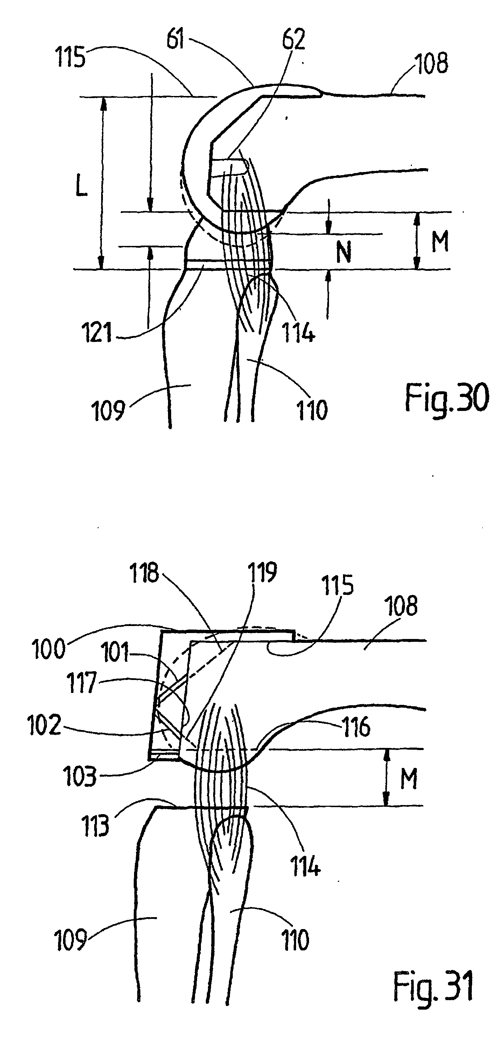

Figures 29 and 31 show a further cutting block 100 with slots 101, 102 and 103 for making the remaining cuts in a patient's femur during resection thereof. Slot 103 is used for making the posterior cut during resection of a patient's femur.

Figure 54 illustrates a square ended key 104 for adjusting the position of compression adjustment member 36 in relation to adjustment knob 30. Its operative square end 105 is adapted to fit in square section recess 39.

The use of the tenser of Figures 1 to 9 and the various ancillary items of equipment will now be explained.

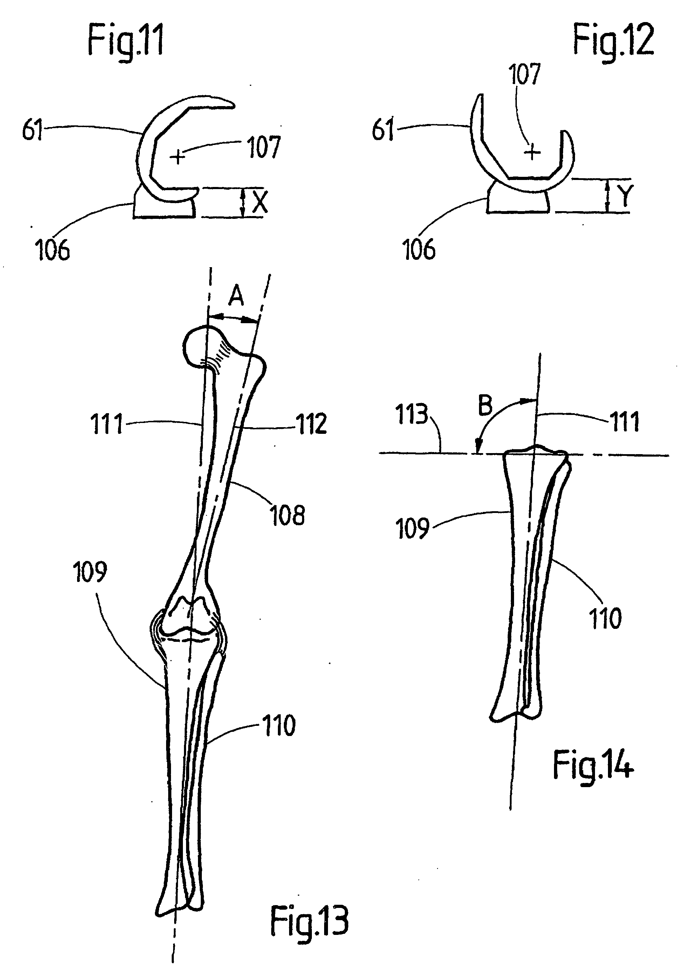

Figure 11 illustrates diagrammatically a femoral component 61 and a tibial insert 106 in the flexed condition of the knee after implantation. The rotational axis of the knee is indicated by reference numeral 107. The distance "X" is typically 18 mm.

Figure 12 illustrates the knee of Figure 11 in the extended position. For proper functioning of the knee the distance "Y" must be the same as the distance "X", i.e. also 18 mm for the example chosen. In addition the collateral ligaments must be correctly tensioned in both the flexed and extended positions of the implanted knee.

In Figure 13 there are illustrated diagrammatically the femur 108, the tibia 109, and the fibula 110 of a healthy erect human. As can be appreciated by inspection of Figure 13, the centre of the hip, the centre of the knee, and the centre of the ankle lie on a straight line 111, while the axis 112 of the femur 108 is offset from line 111 through an angle A, which is typically in the range of from about 3° to about 10°, usually about 7°. This angle A can be measured from X-ray photographs prior to a knee replacement operation.

In a knee replacement operation it is usual for the surgeon to resect first the patient's tibia 109 prior to resecting the patient's femur 108. Figure 14 shows a typical position for a saw cut 113 in resection of the tibia, the angle "B" made between saw cut 113 and the straight line 111, which also corresponds to the axis of the tibia, being substantially equal to 90°.

Figure 15 illustrates the cuts that are made in resecting the tibia 109 and femur 108 during an operation to implant a DBK prosthesis, the knee being illustrated in the flexion position. In Figure 15 the collateral ligament is indicated by reference numeral 114. Having first made the tibial saw cut 113, the surgeon must make a total of five saw cuts during resection of the femur, i.e. an anterior saw cut 115, a posterior saw cut 116, a distal saw cut 117, a first oblique saw cut 118, and a second oblique saw cut 119. Figure 16 shows the same knee but in the extension position. For correct functioning of the knee implant, it is essential that the distances "X" and "Y" indicated in Figures 15 and 16 be essentially equal to one another.

In use of the tenser 1 the surgeon will drill an intramedullary bore for insertion of an intramedullary member 83, as indicated in Figures 17 and 18.

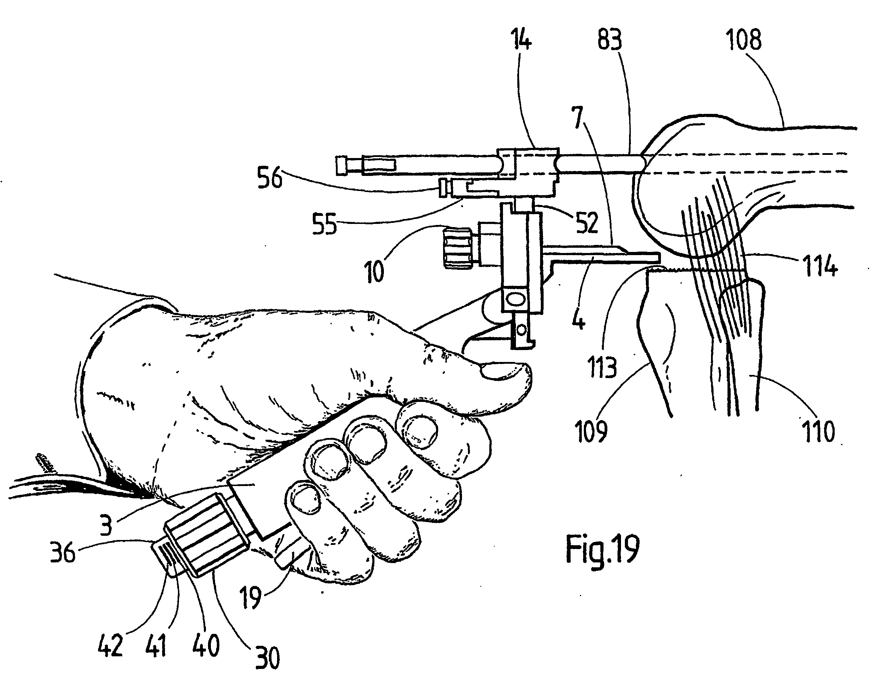

Before the operation the surgeon decides upon the tension to be applied by the tenser 1 to the collateral ligaments and, using the tool 104 of Figure 54, adjusts the position of compression adjustment member 36 relative to adjustment knob 30 until the desired one of the indicia 40, 41 or 42 lies adjacent the posterior end of knob 30. In this way the surgeon selects whether this tension shall be 26 kg, 28 kg, or 30 kg respectively. Then, as shown in Figure 19, having made the tibial cut 113, the surgeon grasps the tenser 1 and squeezes the operating lever extension 19 towards the handle 3 against the action of compression spring 28. As a result of so doing, the surgeon causes the movable paddle member 7 to come into juxtaposition with the fixed paddle member 4. Next the surgeon inserts the intramedullary member 83, which is already positioned in the intramedullary bore, through aperture 15 or aperture 16 depending upon whether a left knee or a right knee is being replaced, with lever 55 having first been set at the appropriate angle, e.g. 7°, corresponding to angle "A" of Figure 13 as determined from previous X-ray photographs.

The tenser 1 is advanced until its paddle members 4 and 7 pass between the resected end 113 of the tibia 109 and the patient's femur 108. Upon releasing the posterior extension 19 of operating lever 8 tension will be applied by compression spring 28 via the paddle members 4 and 7 to the patient's collateral ligaments 114.

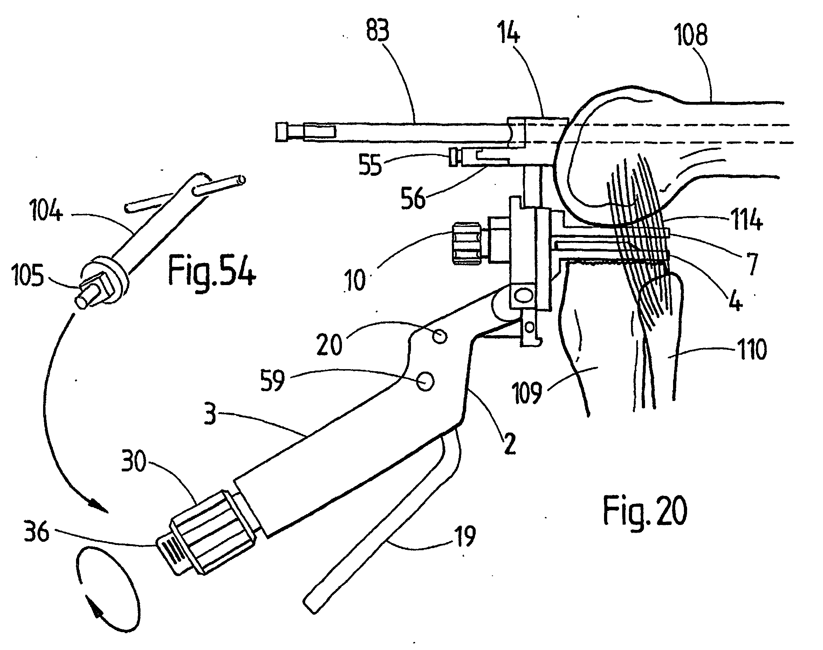

Next the surgeon rotates adjustment knob 30 so as to increase the tension in compression spring 28. As the compression in spring 28 increases so the surgeon will feel the resistance to rotation of adjustment knob 30 increase until the torque required for rotation reaches a value at which the compression in spring 37 is no longer able to prevent grooves 34 from riding over ribs 33. The surgeon can detect that this is happening not only by feel but also because of the audible clicking noise that will result as the grooves 34 ride over ribs 33. Having adjusted adjustment knob 30 in this way, the collateral ligaments will be subjected to the predetermined tension (i.e. 26 kg, 28 kg, or 30 kg). The tenser 1 will now be as shown in Figure 20 with the patient's knee now tensed.

The next step to be taken by the surgeon is to verify the alignment of the patient's leg using the leg alignment member 84 and external adjustment rod holder 85 shown in Figure 21. Figure 22 shows the leg alignment member 84 in position. With the aid of these ancillary items the surgeon can check that the angle at which lever 55 has been set by means of fixed ratchet arc 57 corresponds to the correct angle "A" shown in Figure 22 so that the free end of leg alignment member 84 overlies the centre of the ball 120 of the patient's femur 108.

Alternatively the surgeon can check the leg alignment, with the knee in the extended condition, by use of the leg alignment member 78 of Figure 10, having inserted the peg 82 in bore 59 and locked it in place by means of the spring ball grub screw previously mentioned.

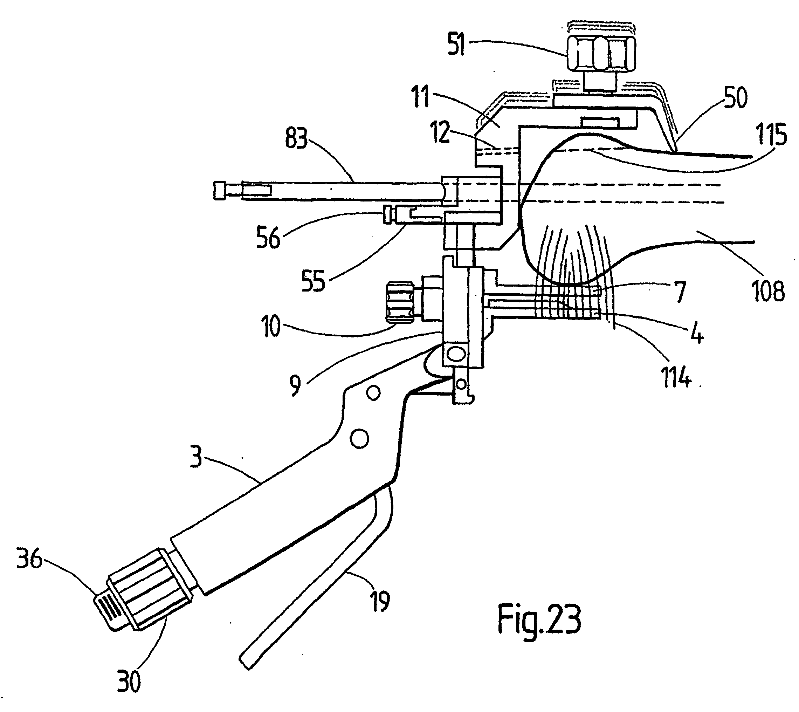

Having checked the leg alignment, the surgeon then removes alignment member 84 and external alignment rod holder 85 and inserts second movable member 11 with its stylus or pointer 50 locked in position using quick locking knob 51 until the tip of stylus or pointer 50 touches the patient's femur 108, as shown in Figure 23. The position of support member 9 is adjusted until indicium 70 is visible in window 71 and then support member 9 is locked to body 2 using locking screw 10 with the surgeon's preliminary choice of tibial insert thickness as selected from the indicia 65, e.g. 9 mm, set against indicium 68 (see Figure 9). With stylus or pointer 50 touching the patient's femur 108, as illustrated in Figure 23, the surgeon can read off against indicia 68 and 69 what will be an appropriate size of femoral component 61, e.g. F3, to use with the selected tibial insert 106.

Figure 23 also shows a potential position for the anterior saw cut 115.

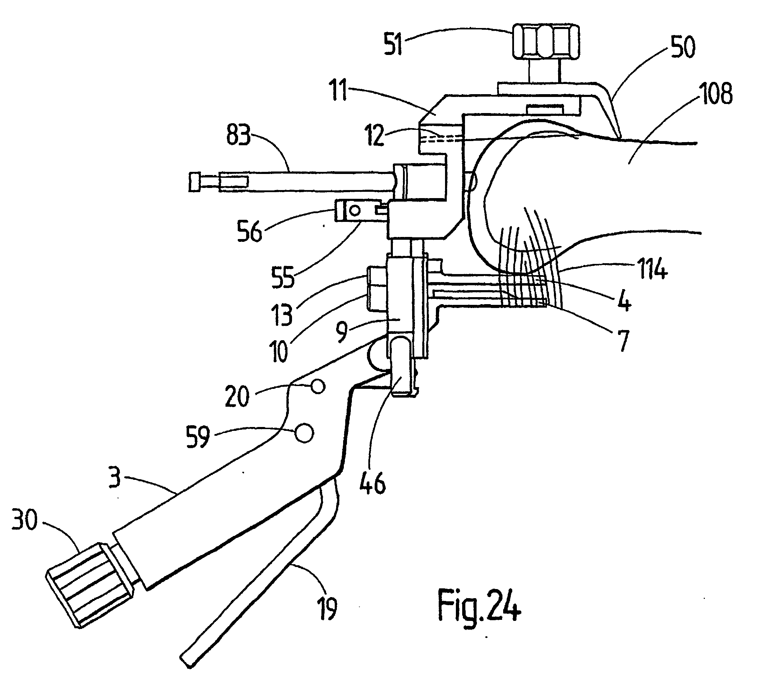

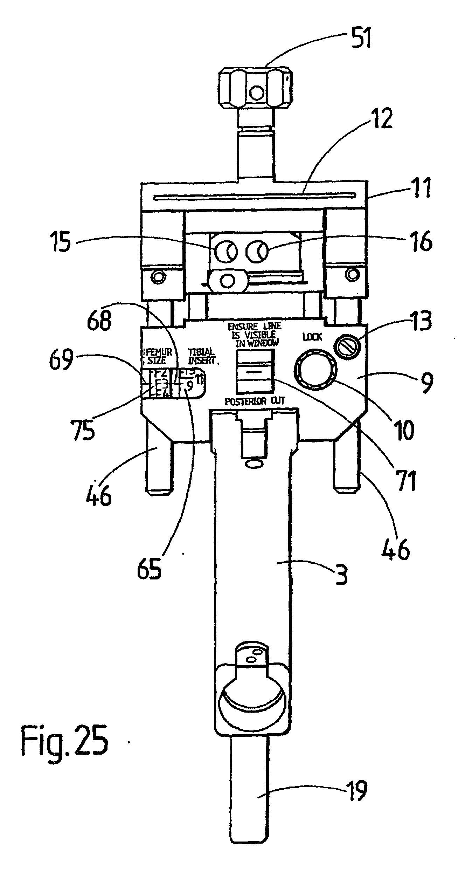

If the indicium 75 for the selected size of femoral component 61 lines up exactly with the indicia 68 and 69, and so does the indicium 65 for the selected thickness, then the surgeon knows that there will be a good fit in the knee implant at the end of the surgical operation. This situation is illustrated in Figures 24 and 25, and particularly in Figure 25 which shows that, for this particular patient, the combination of an F3 femoral component and a 9 mm tibial insert thickness represent a good match for this patient's knee. The surgeon can then proceed to lock second movable member 11 in place in support member 9 using locking screw 13.

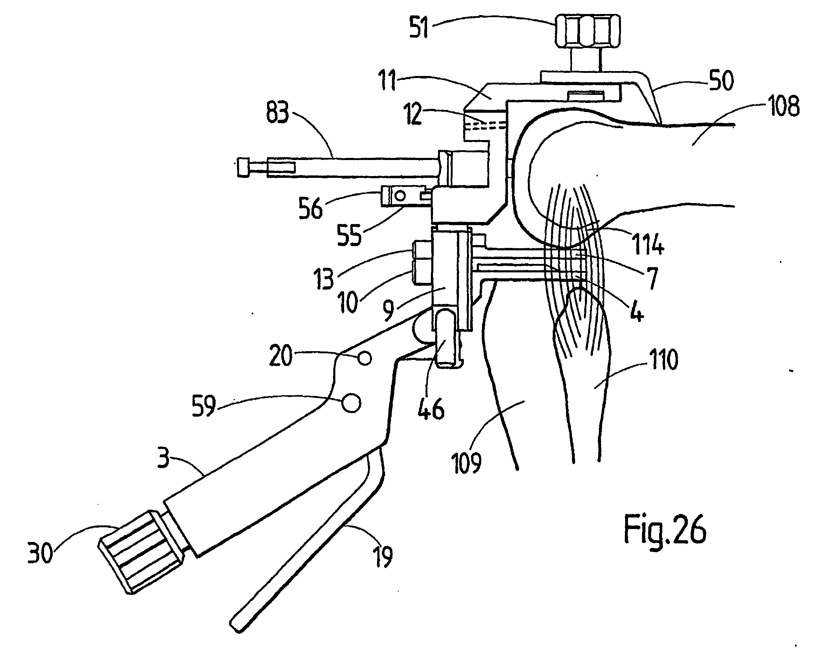

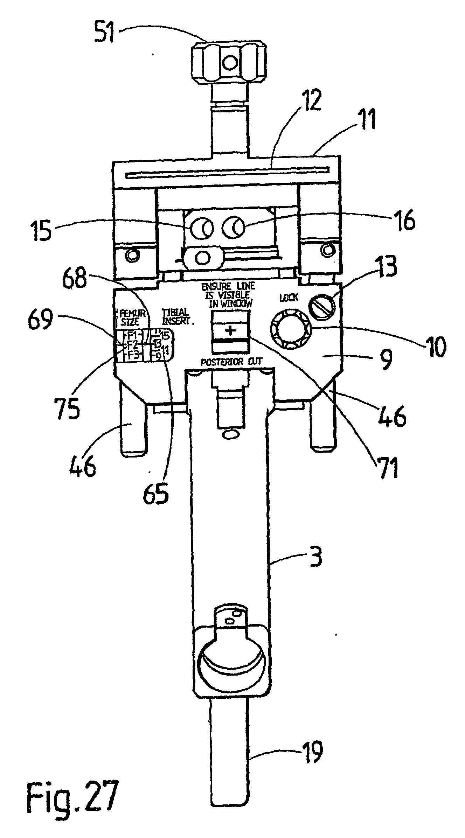

On the other hand, if the indicia 65 and 75 do not line up appropriately, then a further trial selection can be made. For the patient of Figure 26 and 27, for example, a combination of an F2 femoral implant and a 13 mm tibial insert thickness appears to be the best combination.

Figure 28 illustrates a femoral component 61, a tibial insert 106, and a tibial component 121 correctly fitted on a patient's femur 108. The distance "L" between the anterior cut 115 and the tibial cut 113 is fixed when a given tension is applied to the patient's collateral ligaments 114. The tenser 1 allows different ways of filling the space between the anterior cut 115 and the tibial cut 113. As illustrated in Figure 28, one way of filling this space is to use an F3 femoral component 61 and a 9 mm tibial insert 106 so that distance "M" is 18 mm and distance "N" is 9 mm. Thus, as the next steps in the surgical procedure following on from Figures 24 and 25 or Figures 26 and 27, once the surgeon has made the choice of size of femoral component 61 and tibial insert 106, the anterior saw cut 115 can be made using slot 12, followed by the distal saw cut 117, the posterior saw cut 116 and the two oblique saw cuts 118 and 119, using an appropriate size of cutting block 100 with slots 101, 102 and 103 (see Figure 29). In particular posterior saw cut 116 is made at a distance "M" of 18 mm from tibial saw cut 113.

Figure 30 illustrates a knee of a different patient. In this case the surgeon has elected to use an F2 femoral component 62 with a 13 mm tibial insert 106. In this case distance "M" is 22 mm, while distance "N" is 13 mm. A different size of cutting block 100 is accordingly necessary.

Having decided on the size of femoral component 61 and tibial insert 106 to use, the surgeon next removes stylus or pointer 50 and uses the air powered surgical saw 88 to make the anterior saw cut 115 using slot 12 for this purpose, as shown in Figure 32.

Next the surgeon slides block 89 (see Figure 33) by means of its keyway 90 on to arm 49 until it abuts against a stop 122 (see Figure 34). Using holes 91 as a guide the surgeon then drills holes in the patient's femur 108 to facilitate insertion of screws or pins 91 so as to secure block 89 temporarily to the patient's femur 108. Then the intramedullary member 84 can be removed and the tenser 1 removed from the patient's knee by slackening the compression in spring 28 by appropriate rotation of compression adjustment knob 30 and by squeezing posterior extension 19 of operating lever 8 against handle 3. The situation is then as shown in Figure 35. The position of the support member 9 on body 2 should not be altered at this stage.

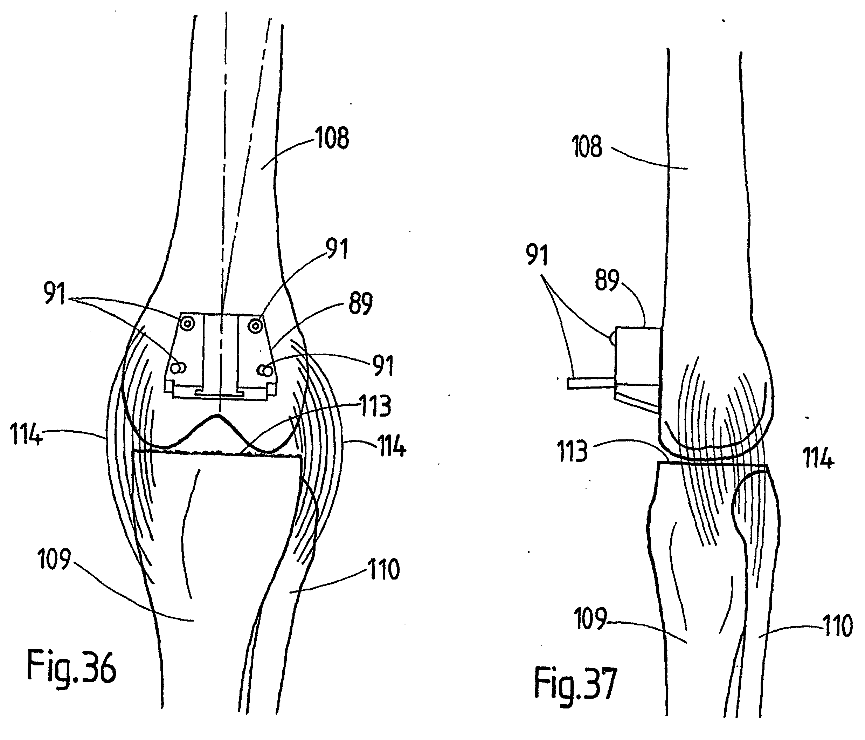

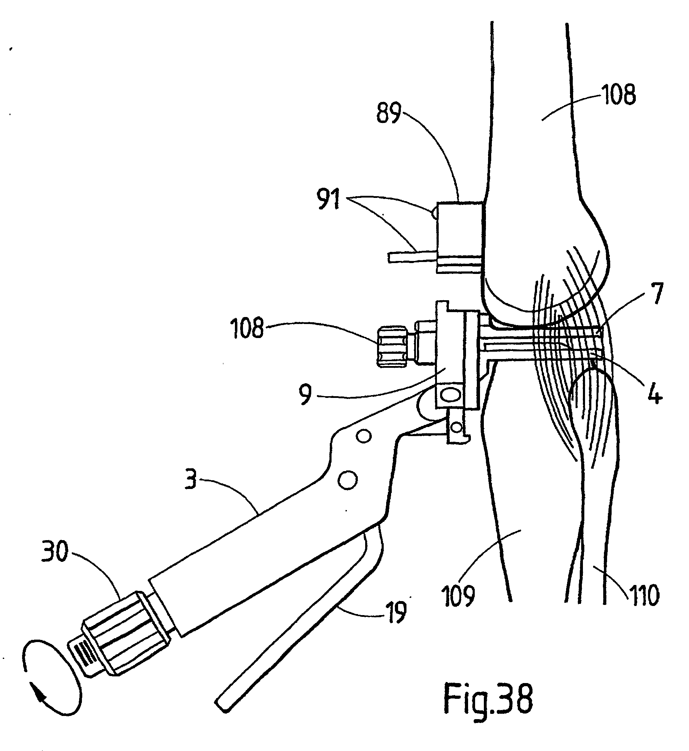

The surgeon then straightens the patient's leg until it is extended as shown in Figures 36 and 37. Next the surgeon will reinsert the tenser 1 as shown in Figure 38 and again operate the adjustment knob 30 to increase the compression in spring 28. Since the position of the compression adjustment member 36 has not been altered, upon the surgeon reapplying compression to spring 28 by rotation of the compression adjustment knob 30 untii the torque limiter operates, the tension applied by the tenser 1 to the collateral ligaments 114 will be the same as before.

Next the surgeon will insert the bar 94 of T-bar attachment 93 (see Figures 39 and 41) into the keyway 90 on block 89 with its crosspiece 95 abutting the top of support member 9 as shown in Figure 40 and lock it in position using the quick release knob 96. The position of the distal cut 117 still to be made is indicated in Figure 43, distance "M" in this case being 18 mm (compare with Figure 28).

Figures 44 to 46 are similar views to those of Figures 40, 42 and 43 for the patient of Figures 30 and 31. In this case distance "M" in Figure 46 is 22 mm (compare with the corresponding distance "M" in Figures 30 and 31.

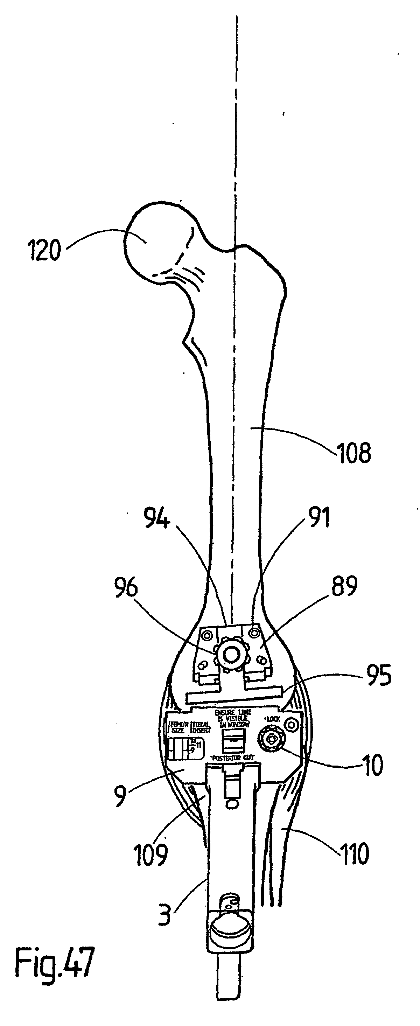

The patient of Figure 47 has a leg misalignment. This is readily apparent because the crosspiece 95 of T-bar attachment 93 is not parallel to the top surface of support member 9. In this case the surgeon can correct this leg misalignment by performing soft tissue release upon the collateral ligaments 114 on the medial side of the patient's knee.

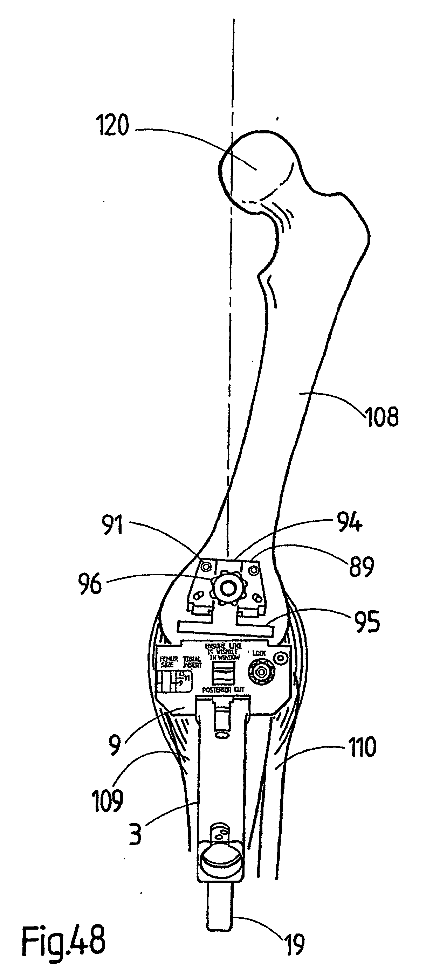

In Figure 48 there is shown the situation for a patient with a leg misalignment in the other direction. In this case the surgeon can correct the misalignment by performing soft tissue release upon the collateral ligaments 114 on the lateral side of the patient's knee.

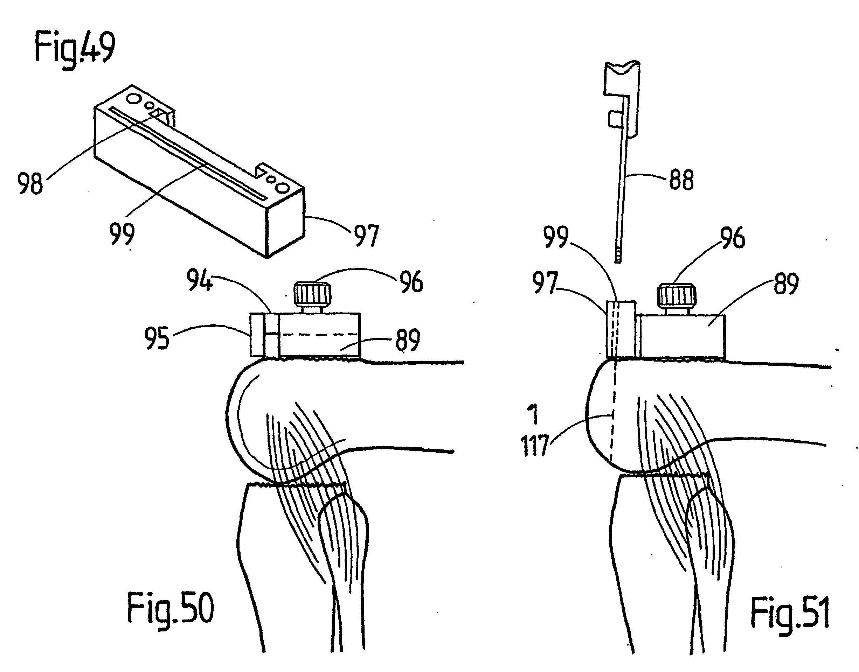

Following any necessary soft tissue release, the surgeon will flex the patient's knee and attach to crosspiece 95 the block 97 for making the distal saw cut 117, this being positioned by means of its keyway 98 on crosspiece 95, as shown in Figures 49 and 50. Using surgical saw 88 the surgeon can then make the distal cut 117 using slot 99, as shown in Figure 51. The distal saw cut block 97, the T-bar attachment 93, and the block 89 are then removed from the patient's leg.

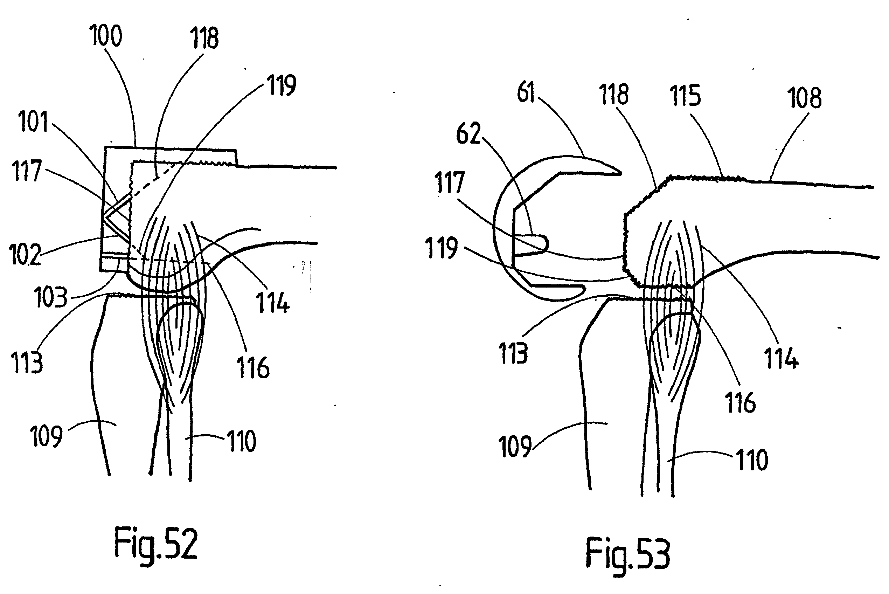

Having made the anterior saw cut 115 and the distal saw cut 117, the surgeon can now fit a block 100 of the appropriate size over the partially resected femur 108 of the patient, as shown in Figure 52, and make the remaining saw cuts, i.e. the posterior saw cut 116 and the two oblique saw cuts 118 and 119, using slots 103, 101 and 102 for the purpose. Finally the femoral component 61 can be fitted to the patient's resected femur 108, as shown in Figure 53, the appropriate bores for the locating pegs 62 on the femoral component 61 having being drilled in the patient's femur 108.

Although the illustrated tenser has been described solely in relation to implantation of a DBK total knee implant, it will be appreciated by persons skilled in the art that the principles of the present invention can readily be adapted for construction of other forms of tenser intended for use with other designs of knee implant.

## Claims

1. A tenser for use during implantation of a knee implant comprising:
a body (2) having a handle (3) at its posterior end and a fixed paddle member (4) at its anterior end, the fixed paddle member (4) having an undersurface (5) defining a plane and adapted for positioning on a resected surface (113) of a tibia (109) which extends substantially orthogonally with respect to the axis of the tibia (109);
a first movable member (6) movably mounted to the body (2) for movement in a direction substantially perpendicular to the plane of the undersurface (5) of the fixed paddle member (4) and having a movable paddle member (7) mounted thereon;
an operating lever (8) mounted in the body (2) and operatively coupled to the first movable member (6) for moving the first movable member (6) relative to the body (2) to move the movable paddle member (7) relative to the fixed paddle member (4) and into engagement with a femur (108) during an operation to implant a knee implant;
a support member (9) adjustably mounted to the body (2) for movement in a direction substantially perpendicular to the plane of the undersurface (5) of the fixed paddle member (4);
first locking means (10) for locking the support member (9) to the body (2);
a second movable member (11) adjustably mounted to the support member (9) for movement in a direction substantially perpendicular to the plane of the undersurface (5) of the fixed paddle member (4), the second movable member (11) being provided with a slot (12) for passage of a surgical saw (88) for making an anterior cut (115) during resection of a femur (108) in preparation for implantation of a femoral component (61);
second locking means (13) for locking the second movable member (11) to the support member (9); and
a third movable member (14) slidably mounted to the body (2) for movement in a direction substantially perpendicular to the plane of the undersurface (5) of the fixed paddle member (4) and having guide means (15, 16) for passage of an intramedullary member (83) for insertion in a prepared intramedullary bore in a femur (108) to be resected.

2. A tenser according to claim 1, in which the operating lever (8) is pivotably mounted in the body (2) and is spring loaded so as to bias the first movable member (6) in an opening direction to cause the movable paddle member (7) to move away from the fixed paddle member (4).

3. A tenser according to claim 2, in which the handle (3) is provided with a longitudinal bore (23, 24) which houses a compression spring (28) which is arranged so as to bias the operating lever (8) in the opening direction.

4. A tenser according to claim 3, in which a piston (25) is disposed in the bore (23, 24) and is interposed between an anterior end of the compression spring (28) and the operating lever (8).

5. A tenser according to claim 3 or claim 4, in which at least a part (23) of the longitudinal bore (23, 24) is threaded, in which a threaded member (26) is threadedly engaged in the threaded part (23) of the bore (23, 24), and in which a posterior end of the compression spring (28) is arranged to bear against the threaded member (26), whereby the compression in the compression spring (28) can be adjusted by moving the threaded member (26) along the threaded part (23) of the longitudinal bore (23, 24).

6. A tenser according to claim 5, in which the handle (3) carries at its posterior end a compression adjustment means (30, 31, 32, 34, 35, 36, 37) adapted for moving the threaded member (26) along the threaded part (23) of the longitudinal bore (23, 24), the compression adjusting means (30, 31, 32, 34, 35, 36, 37) including torque limiting means adapted to limit the torque which can be applied by the compression adjustment means (30, 31, 32, 34, 35, 36, 37) to move the threaded member (26) along the threaded part (23) of the bore (23, 24).

7. A tenser according to claim 6, in which the compression adjustment means (30, 31, 32, 34, 35, 36, 37) comprises an adjustment knob (30) rotatable about a longitudinal axis of the handle (3), an adjustment member (31) in the longitudinal bore (23, 24) and rotatable about the longitudinal axis of the handle (3), the adjustment member (31) being engageable with the threaded member (26) to move the threaded member (26) along the threaded part (23) of the bore (23, 24), and spring loaded clutch means (32, 33, 34, 37) connecting the adjustment knob (30) and the adjustment member (31), whereby upon applying a torque to the adjustment knob (30) in excess of a predetermined torque the spring loaded clutch means (32, 33, 34, 37) prevents further rotation of the adjustment member (31) to increase the compression in the compression spring (28).

8. A tenser according to claim 7, in which the adjustment knob (30) has a compression adjustment member (36) threadedly engaged therewith and in which the spring loaded clutch means (32, 33, 34, 37) comprises a secondary compression spring (37) which is trapped between the compression adjustment member (36) and the adjustment knob (30) and whose compression can be varied by adjusting the position of the compression adjustment member (36) relative to the adjustment knob (30) so as to vary the value of the predetermined torque.

9. A tenser according to claim 8, in which the spring loaded clutch means (32, 33, 34, 37) comprises a collar (32) on the adjustment member (31) having on its posterior side a plurality of radial ribs (33) and in which the adjustment knob (30) is formed with a corresponding plurality of radial grooves (34) on its anterior side for engagement with the radial ribs (33).

10. A tenser according to any one of claims 1 to 9, in which the body (2) bears first indicia (65) corresponding to the thicknesses of available sizes of tibial insert (106), and in which the support member (9) bears a second indicium (68) adapted to indicate against a selected first indicium (65) an appropriate thickness of tibial insert (106) for implantation.

11. A tenser according to any one of claims 1 to 10, in which the support member (9) is provided with a window (71), and in which the body (2) bears a third indicium (70) which is adapted to be visible through the window (71) over a part only of the available range of movement of the support member (9) relative to the body (2) and which is adapted to indicate, when the third indicium (70) is visible in the window (71), that it is appropriate to select a thickness of tibial insert (106).

12. A tenser according to any one of claims 1 to 11, in which the second movable member (11) is provided with fourth indicia (75, 76) corresponding to available sizes of femoral implant (61) and adapted to indicate by reference to an indicium (68, 69) on the support member (9) an appropriate size of femoral implant (61) to use with a selected thickness of tibial insert (106).

13. A tenser according to any one of claims 1 to 12, further including a stylus or pointer device (50) adapted for mounting on the second movable member (11) for contacting a shaft of a femur (108) to be resected during the knee implantation operation.

14. A tenser according to claim 13, in which the second movable member (11) includes an arm (49) adapted to project forwardly over the condyles of a femur (108) to be resected and in which the arm (49) carries a quick release locking device (51) for detachably securing the stylus or pointer (50) to the second movable member (11).

15. A tenser according to any one of claims 1 to 14, in which the third movable member (14) carries an apertured block (54) which is rotatable relative to the third movable member (14) about an axis which is substantially perpendicular to the plane of the undersurface (5) of the fixed paddle member (4) and in which the guide means includes at least one aperture (15, 16) in the apertured block (54) whose axis lies in a plane substantially parallel to the plane of the undersurface (5) of the fixed paddle member (4), whereby the axis of the aperture (15, 16) can be aligned with the axis of an intramedullary bore drilled in a femur (108) to be resected by rotation of the apertured block (54) about its axis.

16. A tenser according to claim 15, in which the third movable member (14) is provided with a ratchet arc (57) substantially coaxial with the axis of rotation of the apertured block (54) and in which a lever (55) with a spring loaded locking device (56) having a detent engageable with the ratchet arc (57) is coupled to the apertured block (57), whereby the apertured block (54) can be locked at a selected position to prevent further rotation about its axis of rotation.

17. A tenser according to any one of claims 1 to 16, further including a leg alignment member (78, 84) which can be operatively coupled to the tenser (1) for checking the alignment of the tenser (1) relative to the axis of a femur (108) to be resected.

18. A tenser according to claim 17, in which the leg alignment member comprises an elongate jogged member (78) and is provided with a transverse peg (82) and in which the body (2) of the tenser (1) is provided with a corresponding transverse bore (59) to receive the peg (82), whereby an anterior portion of the leg alignment member (78) can be positioned forwardly of the tenser (1) along a sagittal plane of the tenser (1) for overlying the ball head (120) of a femur (108) to be resected.

19. A tenser according to claim 18, in which the leg alignment member comprises an elongate member (84) and in which the tenser (1) further includes a support member (85) for the leg alignment member (84) which can be mounted to the support member (9), whereby an anterior portion of the leg alignment member (84) can be positioned forwardly of the tenser (1) along a sagittal plane of the tenser (1) for overlying the ball head (120) of a femur (108) to be resected.

20. A tenser according to any one of claims 1 to 19, further including a removable block (89) which is engageable with the second movable member (11) and is adapted for temporary securement to a femur (108) to be resected.

21. A tenser according to claim 20, in which the removable block (89) is formed with a keyway (90) for engagement with the second movable member (11).

22. A tenser according to claim 20 or claim 21, further including a mounting means (93) for detachable securement to the removable block (89) and for detachably mounting a distal saw cut block (97) thereto, the distal saw cut block (97) being formed with a slot (99) adapted for receipt of a surgical saw (88) to make a distal cut (117) in a femur (108) to be resected.

23. A tenser according to claim 22, in which the mounting means comprises a T-bar attachment (93) including a bar (94) engageable with the removable block (89) and a crosspiece (95) adapted to extend transverse to the axis of a femur (108) to be resected when the mounting means (93) is engaged with the removable block (89) and the removable block (89) is secured to the femur (108) to be resected.

24. A tenser according to claim 23, in which the bar (94) of the T-bar attachment (93) is adapted to be received in a keyway (90) in the removable block (89).

25. A tenser according to any one of claims 1 to 24, in which the movable paddle member (7) is pivotably mounted on the first movable member (6) so as to be pivotable about an axis which is substantially parallel to the plane of the undersurface (5) of the fixed paddle member (4) and lies is a substantially sagittal plane of the tenser (1).

26. A tenser according to any one of claims 1 to 25, further including a saw cut block (100) for applying to a partially resected femur (108) having an anterior saw cut (115) and a distal saw cut (117) formed therein, the saw cut block (100) being provided with slots (101, 102, 103) adapted for making a posterior saw cut (116), a first oblique saw cut (118), and a second oblique saw cut (119).

## Patentansprüche

1. Spanner zur Benutzung während der Implantation eines Knie-Implantats mit
einem Körper (2) mit einem Handgriff (3) an seinem hinteren Ende und einem festen Paddelelement (4) an seinem vorderen Ende, wobei das feste Paddelelement (4) eine von einer Ebene begrenzte Unterseite (5) hat und für die Anordnung auf einer resezierten Oberfläche (113) einer Tibia (109) eingerichtet ist, die sich im wesentlichen rechtwinklig zu der Achse der Tibia (109) erstreckt,
einem ersten beweglichen Element (6), das zur Bewegung in einer Richtung im wesentlichen senkrecht zu der Ebene der Unterseite (5) des festen Paddelelements (4) an dem Körper (2) beweglich angebracht ist und auf dem ein bewegliches Paddelelement (7) angebracht ist,
einem in dem Körper (2) angebrachten Betätigungshebel (8), der mit dem ersten beweglichen Element (6) zu dessen Bewegung relativ zu dem Körper (2) betriebsmäßig gekuppelt ist, um bei einer Operation zur Implantierung eines Knie-Implantats das bewegliche Paddelelement (7) relativ zu dem festen Paddelelement (4) und in Eingriff mit einem Femur (108) zu bewegen,
einem Stützelement (9), das an dem Körper (2) zur Bewegung in einer im wesentlichen senkrechten Richtung zu der Ebene der Unterseite (5) des festen Paddelelements (4) einstellbar angebracht ist,
einer ersten Riegeleinrichtung (10) zur Verriegelung des Stützelements (9) mit dem Körper (2),
einem zweiten beweglichen Element (11), das an dem Stützelement (9) zur Bewegung in einer im wesentlichen senkrechten Richtung zu der Ebene der Unterseite (5) des festen Paddelelements (4) einstellbar angebracht ist, wobei das zweite bewegliche Element (11) mit einem Schlitz (12) für den Durchtritt einer chirurgischen Säge (88) versehen ist, um während der Resektion eines Femurs (108) bei der Vorbereitung zur Implantation eines Femoralbestandteils (61) einen vorderen Schnitt (115) vorzunehmen,
einer zweiten Riegeleinrichtung (13) zur Verriegelung des zweiten beweglichen Elements (11) an dem Stützelement (9), und
einem dritten beweglichen Element (14), das an dem Körper (2) zur Bewegung in einer im wesentlichen senkrechten Richtung zu der Ebene der Unterseite (5) des festen Paddelelements (4) gleitbar angebracht ist und Führungseinrichtungen (15,16) für die Passage eines intramedullären Elements (83) zum Einsatz in eine vorbereitete intramedulläre Bohrung in ein zu resezierendes Femur (108) hat.

2. Spanner nach Anspruch 1, bei dem der Betätigungshebel (8) in dem Körper (2) drehbar angebracht und federbelastet ist, so daß das erste bewegliche Element (6) in einer Öffnungsrichtung vorgespannt ist, um das bewegliche Paddelelement (7) zu veranlassen, sich von dem festen Paddelelement (4) weg zu bewegen.

3. Spanner nach Anspruch 2, bei dem der Handgriff (3) mit einer Längsbohrung (23, 24) versehen ist, in der eine Kompressionsfeder (28) aufgenommen ist, die so eingerichtet ist, daß der Betätigungshebel (8) in der Öffnungsrichtung vorgespannt ist.

4. Spanner nach Anspruch 3, bei dem ein Kolben (25) in der Bohrung (23, 24) zwischen dem vorderen Ende der Kompressionsfeder (28) und dem Betätigungshebel (8) angeordnet ist.

5. Spanner nach Anspruch 3 oder Anspruch 4, bei dem wenigstens ein Teil (23) der Längsbohrung (23, 24) mit einem Gewinde versehen ist, in das ein Gewindeelement (26) eingeschraubt ist und in dem das hintere Ende der Kompressionsfeder (28) angeordnet ist, um gegen das Gewindeelement (26) zu drücken, so daß die Kompression der Kompressionsfeder (28) dadurch eingestellt werden kann, daß man das Gewindeelement (26) längs des Gewindeteils (23) der Längsbohrung (23, 24) bewegt.

6. Spanner nach Anspruch 5, bei dem der Handgriff (3) an seinem hinteren Ende eine Kompressionseinstelleinrichtung (30, 31, 32, 34, 35, 36, 37) trägt, die zur Bewegung des Gewindeelements (26) längs des Gewindeteils (23) der Längsbohrung (23, 24) eingerichtet ist, wobei die Kompressionseinstelleinrichtung (30, 31, 32, 34, 35, 36, 37) eine Drehmoment-Begrenzungseinrichtung enthält, die zur Begrenzung des Drehmoments eingerichtet ist, das durch die Kompressionseinstelleinrichtung (30, 31, 32, 34, 35, 36, 37) angelegt werden kann, um das Gewindeelement (26) längs des Gewindeteils (23) der Bohrung (23, 24) zu bewegen.

7. Spanner nach Anspruch 6, bei dem die Kompressionseinstelleinrichtung (30, 31, 32, 34, 35, 36, 37) einen um die Längsachse des Handgriffs (3) drehbaren Einstellknopf (30), ein in der Längsbohrung (23, 24) befindliches und um die Längsachse des Handgriffs (3) drehbares Einstellelement (31), das mit dem Gewindeelement (26) in Eingriff bringbar ist, um dieses entlang dem Gewindeteil (23) der Bohrung (23, 24) zu bewegen, und den Einstellknopf (30) und das Einstellelement (31) verbindende, federbelastete Kupplungseinrichtungen (32, 33, 34, 37) umfaßt, wodurch beim Anlegen eines Drehmoments an den Einstellknopf (30) über ein vorbestimmtes Drehmoment hinaus die federbelastete Kupplungseinrichtung (32, 33, 34, 37) die weitere Drehung des Einstellelements (31) zur Erhöhung der Kompression der Kompressionsfeder (28) verhindert.

8. Spanner nach Anspruch 7, bei dem der Einstellknopf (30) ein in Gewindeeingriff mit ihm befindliches Kompressionseinstellelement (36) hat und bei dem die federbelastete Kupplungseinrichtung (32, 33, 34, 37) eine sekundäre Kompressionsfeder (37) umfaßt, die zwischen dem Kompressionseinstellelement (36) und dem Einstellknopf (30) eingeschlossen ist und deren Kompression durch Einstellung der Position des Kompressionseinstellelements (36) relativ zu dem Einstellknopf (30) variiert werden kann, um so die Größe des vorbestimmten Drehmoments zu verändern.

9. Spanner nach Anspruch 8, bei dem die federbelastete Kupplungseinrichtung (32, 33, 34, 37) einen Bund (32) auf dem Einstellelement (31) umfaßt, der auf seiner hinteren Seite mehrere radiale Rippen (33) hat, und bei dem auf der Vorderseite des Einstellknopfes (30) eine entsprechende Mehrzahl radialer Vertiefungen (34) für den Eingriff mit den radialen Rippen (33) ausgebildet ist.

10. Spanner nach einem der Ansprüche 1 bis 9, bei dem der Körper (2) erste, den Dicken verfügbarer Tibiaeinsatzgrößen (106) entsprechende erste Anzeigemarken (65) trägt und bei dem das Stützelement (9) eine zweite Anzeigemarke (68) trägt, um gegen eine ausgewählte erste Anzeigemarke (65) eine passende Dicke des Tibiaeinsatzes (106) für die Implantation anzuzeigen.

11. Spanner nach einem der Ansprüche 1 bis 10, bei dem das Stützelement (9) mit einem Fenster (71) versehen ist und bei dem der Körper (2) eine dritte Anzeigemarke (70) trägt, die nur über einen Teil des verfügbaren Bewegungsbereichs des Stützelements (9) relativ zu dem Körper (2) durch das Fenster (71) sichtbar ist und die bei Sichtbarkeit in dem Fenster (71) anzeigt, daß sie zur Wahl einer Dicke des Tibiaeinsatzes (106) geeignet ist.

12. Spanner nach einem der Ansprüche 1 bis 11, bei dem das zweite bewegliche Element (11) mit vierten Anzeigemarken (75, 76) versehen ist, die verfügbaren Größen des Femoralimplantats (61) entsprechen und so eingerichtet sind, durch Bezug auf eine Anzeigemarke (68, 69) auf dem Stützelement (9) eine geeignete Größe des Femoralimplantats (61) zur Verwendung mit einer gewählten Dicke des Tibiaeinsatzes (106) anzuzeigen.

13. Spanner nach einem der Ansprüche 1 bis 12, ferner mit einem Stiftoder Zeigergerät (50), das zur Anbringung an dem zweiten beweglichen Element (11) eingerichtet ist zwecks Kontaktierung eines zu resezierenden Femurschaftes (108) während der Knie-Implantationsoperation.

14. Spanner nach Anspruch 13, bei dem das zweite bewegliche Element (11) einen Arm (49) umfaßt, der nach vorne über die Kondülen eines zu resezierenden Femurs (108) vorstehen kann,und bei dem der Arm (49) ein Schnellfreigabe-Sperrgerät (51) zur lösbaren Befestigung des Stiftes oder Zeigers (50) an dem zweiten beweglichen Element (11) trägt.

15. Spanner nach einem der Ansprüche 1 bis 14, bei dem das dritte bewegliche Element (14) einen mit Öffnungen versehenen Block (54) trägt, der relativ zu dem dritten beweglichen Element (14) um eine Achse drehbar ist, die im wesentlichen senkrecht zu der Ebene der Unterseite (5) des festen Paddelelements (4) steht, und bei dem die Führungseinrichtung wenigstens eine Öffnung (15, 16) in dem mit Öffnungen versehenen Block (54) umfaßt, deren Achse in einer zur Ebene der Unterseite (5) des festen Paddelelements (4) im wesentlichen parallelen Ebene liegt, wodurch die Achse der Öffnung (15, 16) durch Drehung des mit Öffnungen versehenen Blocks (54) um seine Achse mit der Achse einer in ein zu resezierendes Femur (108) gebohrten intramedullären Bohrung ausgerichtet werden kann.

16. Spanner nach Anspruch 15, bei dem das dritte bewegliche Element (14) mit einem Klinkenbogen (57) versehen ist, der im wesentlichen koaxial zu der Drehachse des mit Öffnungen versehenen Blocks (54) ist, und bei dem ein Hebel (55) mit einem federbelasteten Sperrgerät (56), das einen mit dem Klinkenbogen (57) in Eingriff bringbaren Sperrstift hat, mit dem mit Öffnungen versehenen Block (54) gekuppelt ist, wodurch der mit Öffnungen versehene Block (54) in einer ausgewählten Stellung verriegelt werden kann, um eine weitere Drehung um seine Drehachse zu verhindern.

17. Spanner nach einem der Ansprüche 1 bis 16, ferner mit einem Bein-Ausrichtelement (78, 84), das mit dem Spanner (1) betätigungsmäßig gekuppelt werden kann, um die Ausrichtung des Spanners (1) relativ zu der Achse eines zu resezierenden Femurs (108) zu prüfen.

18. Spanner nach Anspruch 17, bei dem das Bein-Ausrichtelement ein längliches abgebogenes Element (78) umfaßt und mit einem Querzapfen (82) versehen ist, und bei dem der Körper (2) des Spanners (1) mit einer entsprechenden Querbohrung (59) zur Aufnahme des Zapfens (82) versehen ist, wodurch ein vorderer Teil des Bein-Ausrichtelements (78) vor dem Spanner (1) entlang seiner Sagittalebene zwecks Überlagerung des Kugelkopfes (120) eines zu resezierenden Femurs (108) positioniert werden kann.

19. Spanner nach Anspruch 18, bei dem das Bein-Ausrichtelement ein längliches Element (84) umfaßt und bei dem der Spanner (1) ferner ein Stützelement (85) für das Bein-Ausrichtelement (84) enthält, das an dem Stützelement (9) angebracht werden kann, wodurch ein vorderer Teil des Bein-Ausrichtelements (84) vor dem Spanner (1) entlang seiner Sagittalebene zwecks Überlagerung des Kugelkopfes (120) eines zu resezierenden Femurs (108) positioniert werden kann.

20. Spanner nach einem der Ansprüche 1 bis 19, ferner mit einem entfernbaren Block (89), der mit dem zweiten beweglichen Element (11) in Eingriff bringbar ist und zur vorübergehenden Befestigung an ein zu resezierendes Femur (108) eingerichtet ist.

21. Spanner nach Anspruch 20, bei dem an dem entfernbaren Block (89) eine Keilnut (90) für den Eingriff mit dem zweiten beweglichen Element (11) ausgebildet ist.

22. Spanner nach Anspruch 20 oder Anspruch 21, ferner mit einer Montageeinrichtung (93) zur lösbaren Befestigung an dem entfernbaren Block (89) und zur lösbaren Anbringung eines distalen Sägeschnittblocks (97) daran, wobei an dem distalen Sägeschnittblock (97) ein Schlitz (99) zur Aufnahme einer chirurgischen Säge (88) ausgebildet ist, um in einem zu resezierenden Femur (108) einen distalen Schnitt (117) auszuführen.

23. Spanner nach Anspruch 22, bei dem die Montageeinrichtung ein T-Schienen-Zusatzteil (93) mit einer mit dem entfernbaren Block (89) in Eingriff bringbaren Schiene (94) und einem Kreuzstück (95) umfaßt, das sich quer zu der Achse eines zu resezierenden Femurs (108) erstrecken kann, wenn die Montageeinrichtung (93) mit dem entfernbaren Block (89) in Eingriff ist und der entfernbare Block (89) an dem zu resezierenden Femur (108) befestigt ist.

24. Spanner nach Anspruch 23, bei dem die Schiene (94) des T-Schienen-Zusatzteils (93) für die Aufnahme in einer Keilnut (90) in dem entfernbaren Block (89) eingerichtet ist.

25. Spanner nach einem der Ansprüche 1 bis 24, bei dem das bewegliche Paddelelement (7) so auf dem ersten beweglichen Element (6) drehbar angebracht ist, daß es um eine Achse drehbar ist, die im wesentlichen parallel zu der Ebene der Unterseite (5) des festen Paddelelements (4) verläuft und in einer im wesentlichen sagittalen Ebene des Spanners (1) liegt.

26. Spanner nach einem der Ansprüche 1 bis 25, ferner mit einem Sägeschnittblock (100) zur Anlage an einem partiell resezierten Femur (108) mit einem vorderen Sägeschnitt (115) und einem darin gebildeten distalen Sägeschnitt (117), wobei der Sägeschnittblock (100) mit Schlitzen (101, 102, 103) versehen ist, die zur Ausführung eines hinteren Sägeschnitts (116), eines ersten schrägen Sägeschnitts (118) und eines zweiten schrägen Sägeschnitts (119) eingerichtet sind.

## Revendications

1. Dispositif tendeur destiné à être utilisé pendant l'implantation d'un implant de genou, comprenant :
un corps (2) ayant une poignée (3) à son extrémité postérieure et un élément en forme de pale fixe (4) à son extrémité antérieure, l'élément en forme de pale fixe (4) présentant une surface inférieure (5) définissant un plan et adaptée à être positionnée sur une surface de résection (113) d'un tibia (109) et qui s'étend sensiblement perpendiculairement par rapport à l'axe du tibia (109) ;
un premier élément mobile (6) monté de façon mobile sur le corps (2) pour un mouvement dans une direction sensiblement perpendiculaire au plan de la surface inférieure (5) de l'élément en forme de pale fixe (4) et comportant un élément en forme de pale mobile (7) monté sur lui-même ;
un levier d'actionnement (8) monté dans le corps (2) et fonctionnellement couplé au premier élément mobile (6) pour déplacer le premier élément mobile (6) par rapport au corps (2) afin de déplacer l'élément en forme de pale mobile (7) par rapport à l'élément en forme de pale fixe (4) et jusqu'en engagement avec un fémur (108) pendant une opération pour implanter un implant de genou ;
un élément de support (9) monté de façon ajustable sur le corps (2) pour un mouvement dans une direction sensiblement perpendiculaire au plan de la surface inférieure (5) de l'élément en forme de pale fixe (4) ;
des premiers moyens de blocage (10) pour bloquer l'élément de support (9) sur le corps (2) ;
un second élément mobile (11) monté de manière ajustable sur l'élément de support (9) pour un mouvement dans une direction sensiblement perpendiculaire au plan de la surface inférieure (5) de l'élément en forme de pale fixe (4), le second élément mobile (11) étant pourvu d'une fente (12) pour le passage d'une scie chirurgicale (88) pour procéder à une découpe antérieure (115) pendant la résection d'un fémur (108) en préparation à l'implantation d'un composant fémoral (61);
des seconds moyens de blocage (13) pour bloquer le second élément de support (11) sur l'élément de support (9); et
un troisième élément mobile (14) monté en coulissement sur le corps (2) pour un mouvement dans une direction sensiblement perpendiculaire au plan de la surface inférieure (5) de l'élément en forme de pale fixe (4) et ayant des moyens de guidage (15, 16) pour le passage d'un élément intramédullaire (83) pour son insertion dans un perçage intramédullaire préparé dans un fémur (108) qui doit subir une résection.

2. Dispositif tendeur selon la revendication 1, dans lequel le levier d'actionnement (8) est monté en pivotement dans le corps (2) et est chargé par un ressort de manière à repousser le premier élément mobile (6) dans une direction d'ouverture pour amener l'élément en forme de pale mobile (7) à se déplacer en éloignement de l'élément en forme de pale fixe (4).

3. Dispositif tendeur selon la revendication 2, dans lequel la poignée (3) est dotée d'un perçage longitudinal (23, 24) qui abrite un ressort de compression (28) qui est agencé de manière à repousser le levier d'actionnement (8) dans la direction d'ouverture.

4. Dispositif tendeur selon la revendication 3, dans lequel un piston (25) est disposé dans le perçage (23, 24) et est interposé entre une extrémité antérieure du ressort de compression (28) et le levier d'actionnement (8).

5. Dispositif tendeur selon l'une ou l'autre des revendications 3 et 4, dans lequel au moins une partie (23) du perçage longitudinal (23, 24) est taraudée, dans lequel un élément fileté (26) est engagé par vissage dans la partie taraudée (23) du perçage (23, 24), et dans lequel une extrémité postérieure du ressort de compression (28) est agencée de manière à porter contre l'élément fileté (26), grâce à quoi la compression dans le ressort de compression (28) peut être ajustée en déplaçant l'élément fileté (26) le long de la partie taraudée (23) du perçage longitudinal (23, 24).

6. Dispositif tendeur selon la revendication 5, dans lequel la poignée (3) porte à son extrémité postérieure des moyens d'ajustement de compression (30, 31, 32, 34, 35, 36, 37) adaptés pour déplacer l'élément fileté (26) le long de la partie taraudée (23) du perçage longitudinal (23, 24), les moyens d'ajustement de compression (30, 31, 32, 34, 35, 36, 37) incluant des moyens de limitation de couple adaptés à limiter le couple qui peut être appliqué par les moyens d'ajustement de compression (30, 31, 32, 34, 35, 36, 37) pour déplacer l'élément fileté (26) le long de la partie taraudée (23) du perçage (23, 24).

7. Dispositif tendeur selon la revendication 6, dans lequel les moyens d'ajustement de compression (30, 31, 32, 34, 35, 36, 37) comprennent un bouton d'ajustement (30) capable de rotation autour d'un axe longitudinal de la poignée (3), un élément d'ajustement (31) dans le perçage longitudinal (23, 24) et capable de rotation autour de l'axe longitudinal de la poignée (3), l'élément d'ajustement (31) pouvant être engagé avec l'élément fileté (26) pour déplacer l'élément fileté (26) le long de la partie taraudée (23) du perçage (23, 24), et des moyens d'embrayage chargés par ressort (32, 33, 34, 37) qui connectent le bouton d'ajustement (30) et l'élément d'ajustement (31), grâce à quoi, lors de l'application sur le bouton d'ajustement (30) d'un couple qui excède un couple prédéterminé, les moyens d'embrayage chargés par ressort (32, 33, 34, 37) empêchent une poursuite de la rotation de l'élément d'ajustement (31) pour augmenter la compression dans le ressort de compression (28).

8. Dispositif tendeur selon la revendication 7, dans lequel le bouton d'ajustement (30) comporte un élément d'ajustement de compression (36) engagé par vissage avec lui-même, et dans lequel les moyens d'embrayage chargés par ressort (32, 33, 34, 37) comprennent un ressort de compression secondaire (37) qui est emprisonné entre l'élément d'ajustement de compression (36) et le bouton d'ajustement (30) et dont la compression peut être variée en ajustant la position de l'élément d'ajustement de compression (36) par rapport au bouton d'ajustement (30) de manière à faire varier la valeur du couple prédéterminé.

9. Dispositif tendeur selon la revendication 8, dans lequel les moyens d'embrayage chargés par ressort (32, 33, 34, 37) comprennent un collier (32) sur l'élément d'ajustement (31) ayant sur son côté postérieur une pluralité de nervures radiales (33), et dans lequel le bouton d'ajustement (30) est formé avec une pluralité correspondante de rainures radiales (34) sur son côté antérieur, pour un engagement avec les nervures radiales (33).

10. Dispositif tendeur selon l'une quelconque des revendications 1 à 9, dans lequel le corps (2) porte des premiers indices (65) correspondant aux épaisseurs des tailles disponibles d'insert de tibia (106), et dans lequel l'élément de support (9) porte un second indice (68) adapté à indiquer, vis-à-vis d'un premier indice choisi (65), une épaisseur appropriée de l'insert de tibia (106) pour l'implantation.

11. Dispositif tendeur selon l'une quelconque des revendications 1 à 10, dans lequel l'élément de support (9) est pourvu d'une fenêtre (71), et dans lequel le corps (2) porte un troisième indice (70) qui est adapté à être visible à travers la fenêtre (71) sur une partie seulement de la plage de mouvement disponible de l'élément de support (9) par rapport au corps (2) et qui est adapté à indiquer, lorsque le troisième indice (70) est visible dans la fenêtre (71), qu'il est approprié pour sélectionner une épaisseur d'insert de tibia (106).

12. Dispositif tendeur selon l'une quelconque des revendications 1 à 11, dans lequel le second élément mobile (11) est pourvu de quatrièmes indices (75, 76) correspondant à des tailles disponibles d'implant fémoral (61) est adaptés à indiquer par référence à un indice (68, 69) sur l'élément de support (9), une taille appropriée d'implant fémoral (61) à utiliser avec une épaisseur choisie d'insert de tibia (106).

13. Dispositif tendeur selon l'une quelconque des revendications 1 à 12, incluant en outre un stylet ou un dispositif pointeur (50) adapté à être monté sur le second élément mobile (11) pour venir en contact avec le fût d'un fémur (108) qui doit subir une résection pendant l'opération d'implantation du genou.

14. Dispositif tendeur selon la revendication 13, dans lequel le second élément mobile (11) inclut un bras (49) adapté à se projeter en avant au-delà des condyles d'un fémur (108) qui doit subir une résection et dans lequel le bras (49) porte un dispositif de blocage à libération rapide (51) pour attacher de façon détachable le stylet ou le pointeur (50) sur le second élément mobile (11).

15. Dispositif tendeur selon l'une quelconque des revendications 1 à 14, dans lequel le troisième élément mobile (14) porte un bloc ajouré (54) qui est capable de tourner par rapport au troisième élément mobile (14) autour d'un axe qui est sensiblement perpendiculaire au plan de la surface inférieure (5) de l'élément en forme de pale fixe (4), et dans lequel les moyens de guidage incluent au moins une ouverture (15, 16) dans le bloc ajouré (54), dont l'axe est situé dans un plan sensiblement parallèle au plan de la surface inférieure (5) de l'élément en forme de pale fixe (4), grâce à quoi l'axe de l'ouverture (15, 16) peut être aligné avec l'axe d'un perçage intramédullaire percé dans un fémur (108) qui doit subir une résection, par rotation du bloc ajouré (54) autour de son axe.

16. Dispositif tendeur selon la revendication 15, dans lequel le troisième élément mobile (14) est pourvu d'un arc à rochet (57) sensiblement coaxial à l'axe de rotation du bloc ajouré (54), et dans lequel un levier (55) avec un dispositif de blocage chargé par ressort (56) possédant un ergot susceptible d'être engagé avec l'arc à rochet (57) est couplé au bloc ajouré (57), grâce à quoi le bloc ajouré (54) peut être bloqué à une position choisie pour empêcher une poursuite de la rotation autour de son axe de rotation.

17. Dispositif tendeur selon l'une quelconque des revendications 1 à 16, incluant en outre un élément d'alignement de jambe (78, 84) qui peut être fonctionnellement couplé au dispositif tendeur (1) pour vérifier l'alignement du dispositif tendeur (1) par rapport à l'axe d'un fémur (108) qui doit subir une résection.

18. Dispositif tendeur selon la revendication 17, dans lequel l'élément d'alignement de jambe comprend un élément coudé allongé (78) et est pourvu d'un pion transversal (82), et dans lequel le corps (2) du dispositif tendeur (1) est pourvu d'un perçage transversal correspondant (59) pour recevoir le pion (82), grâce à quoi une portion antérieure de l'élément d'alignement de jambe (78) peut être positionnée en avant du dispositif tendeur (1) le long d'un plan sagittal du dispositif tendeur (1) pour coiffer la tête sphérique (120) d'un fémur (108) qui doit subir une résection.

19. Dispositif tendeur selon la revendication 18, dans lequel l'élément d'alignement de jambe comprend un élément allongé (84), et dans lequel le dispositif tendeur (1) inclut encore un élément de support (85) pour l'élément d'alignement de jambe (84) qui peut être monté sur l'élément de support (9), grâce à quoi une partie antérieure de l'élément d'alignement de jambe (84) peut être positionnée en avant du dispositif tendeur (1) le long d'un plan sagittal du dispositif tendeur (1) pour coiffer la tête sphérique (120) d'un fémur (108) qui doit subir une résection.

20. Dispositif tendeur selon l'une quelconque des revendications 1 à 19, incluant en outre un bloc amovible (89), qui est susceptible d'être engagé avec le second élément mobile (11) et qui est adapté à être temporairement attaché à un fémur (108) qui doit subir une résection.

21. Dispositif tendeur selon la revendication 20, dans lequel le bloc amovible (89) est formé avec une glissière profilée (90) pour l'engagement avec le second élément mobile (11).

22. Dispositif tendeur selon l'une ou l'autre des revendications 20 et 21, incluant en outre des moyens de montage (93) pour attacher de façon détachable sur le bloc amovible (89) et pour monter de façon détachable sur celui-ci un bloc de coupe à scie distale (97), le bloc de coupe à scie distale (97) étant formé avec une fente (99) adaptée à recevoir une scie chirurgicale (88) pour pratiquer une coupe distale (117) dans un fémur (108) qui doit subir une résection.

23. Dispositif tendeur selon la revendication 22, dans lequel les moyens de montage comprennent une attache (93) à barre en forme de T, incluant une barre (94) susceptible d'être engagée avec le bloc amovible (89) et une traverse (95) adaptée à s'étendre transversalement à l'axe d'un fémur (108) qui doit subir une résection quand les moyens de montage (93) sont engagés avec le bloc amovible (89) et que le bloc amovible (89) est fixé sur le fémur (108) qui doit subir une résection.

24. Dispositif tendeur selon la revendication 23, dans lequel la barre (94) de l'attache à barre en forme de T (93) est adaptée à être reçue dans une glissière profilée (90) dans le bloc amovible (89).

25. Dispositif tendeur selon l'une quelconque des revendications 1 à 24, dans lequel l'élément en forme de pale mobile (7) est monté en pivotement sur le premier élément mobile (6) de manière à pouvoir pivoter autour d'un axe qui est sensiblement parallèle au plan de la surface inférieure (5) de l'élément en forme de pale fixe (4), et qui est disposé dans un plan sensiblement sagittal du dispositif tendeur (1).

26. Dispositif tendeur selon l'une quelconque des revendications 1 à 25, incluant en outre un bloc de coupe à scie (100) destiné à être appliqué sur un fémur ayant subi une résection partielle (108), possédant une coupe de scie antérieure (115) et une coupe de scie distale (117) formées à l'intérieur, le bloc de coupe à scie (100) étant pourvu de fentes (101, 102, 103) adaptées à pratiquer une coupe de scie postérieure (116), une première coupe de scie en oblique (118), et une seconde coupe de scie en oblique (119).
